# EUROPÄISCHE PATENTANMELDUNG

(11) **EP 1 724 249 A2**
(43) Veröffentlichungstag der Anmeldung: **22.11.2006**
(21) Anmeldenummer: 06111749.5
(22) Anmeldetag: 27.03.2006
(51) Int. Cl.: C07C 2/08, C07C 11/02, C07C 7/08, C07C 11/08

(54) **Verfahren zur Oligomerisierung von Butenen**

(30) Priorität: 21.05.2005 DE 102005023549
(71) Anmelder: Oxeno Olefinchemie GmbH, 45772 Marl (DE)
(72) Erfinder: Kerker, Lothar, 48249 Dülmen (DE); Malzkorn, Rainer, 36695 Mobile AL Alabama (US); Höper, Frank, 45721 Haltern am See (DE); Röttger, Dirk, 45657 Recklinghausen (DE); Rix, Armin, 45770 Marl (DE)

(57) **Zusammenfassung**

Die Erfindung betrifft ein Verfahren zur Oligomerisierung von Butenen, bei dem ein Strom, der überwiegend Butene enthält, welcher durch Abtrennung aus einem Strom von Kohlenwasserstoffen mit geringerem Gehalt an Butenen, Wäsche und Trocknung erhalten wurde, der Oligomerisierung zugeführt wird.

## Beschreibung

Die Erfindung betrifft ein Verfahren zur Oligomerisierung von Butenen aus einem Strom, der ganz oder teilweise aus einem Kohlenwasserstoffstrom besteht, der überwiegend Butene enthält und welcher durch Abtrennung aus einem Strom von Kohlenwasserstoffen mit geringerem Gehalt an Butenen, Wäsche und anschließende Trocknung erhalten wurde.

Die Oligomerisierung von Olefinen, insbesondere von C₄-Olefinen, stellt ein industriell häufig angewendetes Verfahren dar. Durch die Oligomerisierung von C₄-Olefinen werden insbesondere Olefine mit acht, zwölf, sechzehn oder zwanzig Kohlenstoffatomen erhalten. Diese Olefine werden z. B. zur Herstellung von Weichmacheralkoholen (C₉- oder C₁₃₋Alkohole) oder von Alkoholen (C₁₃-, C₁₇- oder C₂₁-Alkohole) zur Herstellung von Waschmittelrohstoffen verwendet.

Es sind verschiedene Oligomerisierungsverfahren bekannt. Im Prinzip gibt es drei Verfahrensvarianten. Lange bekannt ist die Oligomerisierung an sauren Katalysatoren (Verfahren A), wobei technisch z. B. Zeolithe oder Phosphorsäure auf Trägem eingesetzt werden. Hierbei werden Isomerengemische von verzweigten Olefmen erhalten, die im Wesentlichen Dimethylhexene darstellen (WO 92/13818). Ein ebenfalls weltweit ausgeübtes Verfahren ist die Oligomerisierung mit löslichen Ni-Komplexen, bekannt als DIMERSOL-Verfahren (Verfahren B) (B. Cornils, W.A. Herrmann, Applied Homogenous Catalysis with Organometallic Compounds, Seite 261-263,Verlag Chemie 1996). Schließlich ist noch die Oligomerisierung an Nickel-Festbett-Katalysatoren zu erwähnen, wie z. B. das Verfahren der OXENO Olefinchemie GmbH. Das Verfahren hat Eingang in die Literatur als OCTOL-Prozess (Verfahren C) (Hydrocarbon Process., Int. Ed. (1986) 65 (2. Sect.1), Seiten 31 bis 33) gefunden und kann auch DE 39 14 817 sowie EP 1 029 839 entnommen werden.

Die bekannten Oligomerisierungsverfahren haben den Nachteil, dass der Olefinumsatz in der Oligomerisierungsstufe nicht vollständig ist, und häufig sogar nur in einem Bereich von ca. 50 % liegt. Zur Verbesserung des Umsatzes werden entweder mehrere Oligomerisierungsstufen hintereinandergeschaltet oder die Oligomerisierungsstufe wird Schlaufenfahrweise bzw. mit (Teil-)Rückführung des Produktstromes durchgeführt. Diese Verfahren haben den Nachteil, dass große Stoffströme bewegt werden müssen. Dies ist insbesondere dann nachteilig, wenn nicht allein die umzusetzenden Butene, sondern auch noch der Oligomerisierung nicht zugängliche Butane im Oligomerisierungsgemisch vorhanden sind.

Ebenfalls nachteilig an den im Stand der Technik bekannten Oligomerisierungsverfahren ist die Empfindlichkeit der eingesetzten Katalysatoren gegenüber im Einsatzstoff vorhandenen Katalysatorgiften. Die Katalysatorgifte sind je nach eingesetztem Oligomerisierungsprozess (Variante A, B oder C) unterschiedlich. Verfahren zu Entfernung von Katalysatorgiften durch den Einsatz von Adsorptionsmitteln sind beispielsweise in DE 19845857 und DE 3914817 beschrieben. Diese kommen insbesondere beim Einsatz von Übergangsmetallkatalysatoren, meistens auf Nickel-Basis, zum Tragen.

Es war Aufgabe der vorliegenden Erfindung ein Verfahren bereitzustellen, mit welchem die Nachteile der Verfahren des Standes der Technik weitestgehend vermieden werden können. Insbesondere war Aufgabe der vorliegenden Erfindung ein Verfahren zur Oligomerisierung von Butenen bereitzustellen, bei dem der eingesetzte Rohstoff eine ausreichende Butenkonzentration aufweist und gleichzeitig weitestgehend frei von Katalysatorgiften ist.

Überraschenderweise wurde gefunden, dass es durch die Kombination einer Buten/Butan-Abtrennung auf Basis einer Extraktivdestillation mit einer Oligomerisierung auf einfache Weise möglich ist, die Nachteile des Standes der Technik zu vermeiden. Insbesondere kann auch der Anteil an Katalysatorgiften reduziert werden, wenn der an Butenen angereicherte Strom aus der Extraktivdestillation vor der Trocknung für den Einsatz in der Oligomerisierung mit Wasser oder einer wässrigen Lösung gewaschen wird.

Gegenstand der vorliegenden Erfindung ist deshalb ein Verfahren zur Oligomerisierung von Butenen in Gegenwart eines Übergangsmetallkatalysators, bei dem als Einsatzstoff ein Kohlenwasserstoffstrom eingesetzt wird, der ganz oder teilweise aus einem Kohlenwasserstoffstrom (1) besteht, der überwiegend Butene enthält und der durch Abtrennung aus einem Strom von Kohlenwasserstoffen (2) mit geringerem Gehalt an Butenen erhalten wurde, welches dadurch gekennzeichnet ist, dass der Kohlenwasserstoffstrom (1) durch
a) extraktive Destillation eines gesättigte und ungesättigte C₄-Kohlenwasserstoffe enthaltenden Stroms (2) mit einem polaren Extraktionsmittel unter Erhalt einer an gesättigten Kohlenwasserstoffen angereicherten Kopffraktion (3) und einer an ungesättigten Kohlenwasserstoffen angereicherten und das polare Extraktionsmittel enthaltenden Sumpffraktion (4),
b) destillative Trennung der Sumpffraktion (4) in eine Kopffraktion (5), die als ungesättigte Kohlenwasserstoffe Butene enthält und eine Sumpffraktion (6), die das polare Extraktionsmittel enthält,
c) Waschen zumindest eines Teils der Kopffraktion (5) mit Wasser oder einer wässrigen Lösung und
d) Trocknung des in Schritt c) behandelten Teils der Kopffraktion (5) unter Erhalt eines weitgehend wasserfreien butenhaltigen Kohlenwasserstoffstroms (1),
erhalten wird.

Das erfindungsgemäße Verfahren hat den Vorteil, dass auf einfache Weise in Kohlenwasserstoffströmen enthaltene Butene mit hoher Ausbeute zu Oligomeren umgesetzt werden können. Durch die Abtrennung der gesättigten Kohlenwasserstoffe von den Butenen werden die in der anschließenden Oligomerisierung zu behandelnden Stoffströme deutlich kleiner, wodurch Energie- und Materialkosten eingespart werden können.

Das erfindungsgemäße Verfahren hat außerdem den Vorteil, dass in der Oligomerisierung der eingesetzte Übergangsmetallkatalysator eine relativ lange Standzeit aufweist, da Katalysatorgifte durch die Wäsche verringert werden. Dies ist insbesondere bei heterogenen Katalysatoren von Bedeutung. Bei diesen sollte die Standzeit ausreichen, um in Festbettreaktoren (z. B. adiabatischen Festbettreaktoren, Rohbündelreaktoren) arbeiten zu können.

Die für den Betrieb des Verfahrens notwendige Energie kann durch geeignete Prozessführung, wie sie in bevorzugten Ausführungsformen des erfindungsgemäßen Verfahren beansprucht werden, reduziert werden. Wird in dem erfindungsgemäßen Verfahren die Sumpffraktion aus Stufe b) zumindest teilweise als Extraktionsmittel in die Stufe a) eingespeist und die in der Sumpffraktion enthaltene Wärmeenergie in einem Wärmetauscher genutzt, um den Zulauf (Feed) zur Destillationskolonne der Stufe b) zu erwärmen, so können die notwendigen Energiemengen beim Betrieb des Verfahrens gegenüber herkömmlichen Verfahren deutlich reduziert werden.

Durch die Aufheizung des der Stufe b) zugeführten Stroms kann außerdem bereits eine teilweise Verdampfung der Bestandteile des Stroms erreicht werden, wodurch eine verbesserte Trennung in der Stufe b) erreicht werden kann. Insbesondere weist diese Ausführungsform des erfindungsgemäßen Verfahrens den Vorteil auf, dass die Investitionskosten geringer gehalten werden können, da auf Pumpen und Sammelböden verzichtet werden kann und die Kolonne der Stufe b) mit einer geringeren Kolonnenhöhe gebaut werden kann.

Der Einsatz von Dekantem in einer bevorzugten Ausführungsform des erfindungsgemäßen Verfahrens hat den Vorteil, dass die Extraktionsmittel weitestgehend aus den zu trennenden Kohlenwasserstoffen nach der Auftrennung in ungesättigte und gesättigte Kohlenwasserstoffe wieder entfernt werden können. Durch zumindest teilweise Rückführung in die Extraktionsstufe a) oder die Trennstufe b) wird der Verbrauch an Lösungsmittel gesenkt und die Zusammensetzung des Extraktionsmittelgemisches bleibt auch in kontinuierlich betriebenen Verfahren weitestgehend konstant. Der Einsatz der erfindungsgemäß bevorzugten Dekantertechnik in Schritt b) ist daher sinnvoll, obwohl die ungesättigten Kohlenwasserstoffströme zu einer weiteren Reinigung in einer Wasserwäsche nachbehandelt werden. Durch die Wäsche mit Wasser oder wässrigen Lösungen gelingt es, den Gehalt an organischem Extraktionsmittelbestandteil so weit zu reduzieren, dass der butenhaltige Strom nach der Trocknung in der Oligomerisierung eingesetzt werden kann.

Auch zur Nachreinigung der in Schritt a) erhaltenen Kopffraktion (3) kann eine Wäsche mit Wasser oder wässrigen Lösungen genutzt werden. Dies kann für einige Anwendungen der erhaltenen Butane oder Butene notwendig sein. Aus der Kopffraktion (3) sind beispielsweise nach weiterer Aufarbeitung Butane erhältlich, die Verwendung als Treibmittel finden können.

In ihnen verursachen Reste von Extraktionsmittel schon in geringen Konzentrationen einen wahrnehmbaren unangenehmen Geruch, der die Verwendung der Butane als Treibmittel verhindert. Eine Abtrennung der Extraktionsmittelanteile in den erfindungsgemäßen Dekantem vor einer Wasserwäsche kann zudem einem Eintrag von Extraktionsmittel in die Wasserwäschen entgegengewirkt werden.

Nachfolgend wird das erfindungsgemäße Verfahren beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind im nachfolgenden Text Bereiche bzw. Vorzugsbereiche angegeben, so sollen auch alle in diesen Bereichen liegenden, theoretisch möglichen Teilbereiche und Einzelwerte zum Offenbarungsgehalt der vorliegenden Erfindung gehören, ohne dass diese aus Gründen der besseren Übersichtlichkeit explizit genannt worden sind.

Die Auftrennung von Butenen und Butanen ist in der Fachliteratur bekannt. Da eine einfache destillative Trennung aufgrund der Siedepunktsdifferenzen nicht technisch sinnvoll durchzuführen ist, werden hierzu meist Extraktivdestillationen mit polaren Lösemitteln eingesetzt. So beschreibt EP 501 848 die Auftrennung eines von Butadien befreiten C₄-Schnitts durch Extraktiv-Destillation mit einem Extraktionsmittel wie n-Methylpyrrolidon (NMP) oder Dimethylformamid (DMF) in drei Stufen: In der ersten Stufe wird das Einsatz C₄₋Kohlenwasserstoffgemisch mit dem Extraktionsmittel in einer Extraktivdestillations-kolonne versetzt. Hierbei lösen sich die olefinischen Bestandteile im Extraktionsmittel, so dass die schlechter löslichen aliphatischen Bestandteile abgetrennt werden können. Zur weiteren Trennung bzw. zur Rückgewinnung des Extraktionsmittels wird dann unter einem Druck von 0,4 bis 0,5 MPa eine teilweise Desorption der Butene aus dem Extrakt durchgeführt. Zur restlichen Rückgewinnung des Extraktionsmittels erfolgt anschließend ein Auskochen des Extrakts bei Normaldruck und einer Temperatur von 140 bis 170 °C.

In JP 692 876 ist die Verwendung von Dimethylformamid als polares Extraktionsmittel zur Buten-/Butantrennung offengelegt. Weiterhin beschreibt diese Druckschrift, dass nach der extraktiven Destillation und der Abtrennung der aliphatischen Bestandteile aus dem EinsatzKohlenwasserstoffgemisch der größte Teil des polaren Extraktionsmittels durch eine Desorptionsstufe bei 1 bis 2 Atmosphären unter Rückführung des größten Teils des Extraktionsmittels durchgeführt wird. Die butenhaltige Fraktion wird in einer Reinigungsstufe bei erhöhtem Druck von 1 bis 15 Atmosphären von den Butenen befreit; das so erhaltene reine Extraktionsmittel wird wiederum in die Extraktionsdestillationsstufe zurückgeführt. Das Extraktionsmittel enthält gemäß den Beispielen noch große Anteile von Butenen, die mit dem Extraktionsmittel zurückgeführt, d. h. im Kreis geführt werden. Dies ist energetisch und ökonomisch ungünstig.

Bender et al beschreiben in Bender, D.; Lindner, A.; Schneider, K.J; Volkamer, K.; BASF ENTWICKLUNGSARBEITEN AM BUTADIENVERFAHREN DER BASF; Erdoel Kohle, Erdgas, Petrochem. (1981) 34(8), 343, den Einsatz von wasserhaltigen Extraktionsmitteln in Verfahren zur Trennung von gesättigten und ungesättigten Kohlenwasserstoffen. Dadurch kann die Selektivität des Extraktionsmittels optimiert werden. Technisch kann eine Erhöhung des Siededrucks des Extraktionsmittels durch Zugabe von Wasser vorteilhaft sein, um beispielsweise mit normalem Kühlwasser/Flusswasser ohne Brüdenkompression die Brüden kondensieren zu können.

Der Einsatz von Extraktionsmitteln, die aus Mischungen eines oder mehrerer polarer organischer Lösungsmittel und Wasser bestehen, führt zu dem Problem, dass sowohl am Kopf der Extraktions- als auch der Ausgaserkolonne Teile des Extraktionsmittels anfallen. Dabei handelt es sich größtenteils um Wasser, in dem meist noch geringe Anteile der organischen Lösungsmittel enthalten sind. Bei den bekannten Verfahren werden diese Extraktionsmittelanteile mit den C₄-Kohlenwasserstoffen aus dem Prozess ausgetragen, was bei einem kontinuierlichen Betrieb zu einer Änderung der Zusammensetzung des Extraktionsmittels führt.

Die Offenlegung DE 102 42 923 beschreibt eine Verfahrensvariante zur Trennung von Butenen und Butanen, die dieses Problem teilweise löst. Dabei wird die Abtrennung der Butene vom polaren, wasserhaltigen Extraktionsmittel in zwei Stufen durchgeführt. Zur Abtrennung der wässrigen Phase wird eine weitere Stufe benötigt. In ihr fallen die Butene als Sumpffraktion an, am Kolonnenkopf wird in einem Dekanter eine wässrige Phase erhalten, die in die erste Stufe der Trennung von Butenen und Extraktionsmittel zurückgeführt wird.

In der Offenlegung DE 2 359 300 wird der Einsatz von Dekantem zur Abtrennung einer wässrigen Phase bei einem Verfahren zur Gewinnung von gesättigten Kohlenwasserstoffen aus Kohlenwasserstoffgemischen beschrieben. Die extraktive Destillation wird dabei unter Rückfluss von Wasser vom Kopf der Kolonne her durchgeführt. Für die dabei erhaltenen gesättigten Kohlenwasserstoffe wird eine Größenordnung an polaren Verunreinigungen von 100 ppm angegeben.

In DE 102 19 375 wird ein Verfahren zur Gewinnung von Butenen aus einem C₄-Schnitt beschrieben, in welchem versucht worden ist, die Energieausnutzung in dem Verfahren zu verbessern. Die in dem Sumpfprodukt der Stufe der Abtrennung des Extraktionsmittels (Ausgaszone) enthaltene Wärme wird genutzt, um einen Teilstrom, der aus der Ausgaszone abgezogen wird, zu erwärmen, bevor dieser in die Ausgaszone zurückgeführt wird.

Im erfindungsgemäßen Verfahren wird aufbauend auf diesen bekannten Verfahren zur Trennung von Butenen und Butanen ein Kohlenwasserstoffstrom, der zur Oligomerisierung eingesetzt werden soll, zunächst durch Extraktivdestillation an Butenen angereichert und anschließend an evtl. vorhandenen Katalysatorgiften abgereichert, bevor der Kohlenwasserstoffstrom der Oligomerisierung zugeführt wird.

Das erfindungsgemäße Verfahren zur Oligomerisierung von Butenen in Gegenwart eines Übergangsmetallkatalysators, vorzugsweise eines heterogenen Übergangsmetallkatalysators, bei dem als Einsatzstoff ein Kohlenwasserstoffstrom eingesetzt wird, der ganz oder teilweise aus einem Kohlenwasserstoffstrom (1) besteht, der überwiegend Butene enthält und der durch Abtrennung aus einem Strom von Kohlenwasserstoffen (2) mit geringerem Gehalt an Butenen erhalten wurde, zeichnet sich dadurch aus, dass der Kohlenwasserstoffstrom (1) durch
a) extraktive Destillation eines gesättigte und ungesättigte C₄-Kohlenwasserstoffe enthaltenden Stroms (2) mit einem polaren Extraktionsmittel unter Erhalt einer an gesättigten Kohlenwasserstoffen angereicherten Kopffraktion (3) und einer an ungesättigten Kohlenwasserstoffen angereicherten und das polare Extraktionsmittel enthaltenden Sumpffraktion (4),
b) destillative Trennung der Sumpffraktion (4) in eine Kopffraktion (5), die als ungesättigte Kohlenwasserstoffe Butene enthält und eine Sumpffraktion (6), die das polare Extraktionsmittel enthält,
c) Waschen zumindest eines Teils der Kopffraktion (5) mit Wasser oder einer wässrigen Lösung und
d) Trocknung des in Schritt c) behandelten Teils der Kopffraktion (5) unter Erhalt eines weitgehend wasserfreien butenhaltigen Kohlenwasserstoffstroms (1),
erhalten wird.

Die Oligomerisierung der im Einsatzkohlenwasserstoffstrom enthaltenen Butene kann nach einem der aus dem Stand der Technik bekannten Verfahren durchgeführt werden. Bevorzugt werden im erfindungsgemäßen Verfahren als Oligomerisierungsverfahren die Verfahren eingesetzt, die unter den Namen Dimersol-Prozess (B. Comils, W. A. Herrmann, Applied Homogeneous Catalysis with Organometallic Compounds, Seiten 261 bis 263, Verlag Chemie, 1996) oder Octol-Prozess (EP 1 029 839, DE 39 14 817, Hydrocarbon Processing, International Edition (1986), 65(2, Sect. 1), 31 bis 33) bekannt sind. Auf die hier zitierten Schriften sei ausdrücklich verwiesen und deren Offenbarung soll zum Offenbarungsgehalt der vorliegenden Erfindung gehören. Im Dimersol-Prozess kommt ein gelöster Nickel-Katalysator zum Einsatz. Im Octol Prozess können heterogene Katalysatoren, die Übergangsmetalle, hauptsächlich Nickel, enthalten können, eingesetzt werden. Im Rahmen der vorliegenden Erfindung werden für die Oligomerisierung besonders bevorzugt heterogene Nickelkatalysatoren eingesetzt, insbesondere Nickelträgerkatalysatoren, wie beispielsweise Nickel auf Siliciumdioxid oder Nickel auf Siliciumdioxid-Aluminiumoxid. Weitere heterogene Nickelkatalysatoren werden z. B. in US 5,169,824, EP 1 268 370 und WO 95/14647 und in der dort zitierten Literatur beschrieben. Der Nickelgehalt der Katalysatoren liegt typischerweise bei 1 bis 25 Massen-%.

Die Oligomerisierung in dem erfindungsgemäßen Verfahren kann insbesondere wie nachfolgend beschrieben durchgeführt werden. Bei diesem Verfahren werden die in dem Kohlenwasserstoffstrom, der zumindest teilweise aus dem Kohlenwasserstoffstrom (1) besteht, enthaltenen Butene an einem nickelhaltigen Katalysator bei Temperaturen von 0 bis 200 °C und bei Drücken von 0,1 bis 7 MPa oligomerisiert. Die Oligomerisierung kann sowohl in Gegenwart homogener als auch in Gegenwart heterogener Katalysatoren vorgenommen werden. Bevorzugt erfolgt die Oligomerisierung an einem heterogenen nickelhaltigen Katalysator, besonders bevorzugt an einem nickelhaltigen Katalysatorfestbett und ganz besonders bevorzugt an einem nickel-, silizium- und aluminiumhaltigen Katalysatorfestbett. Die Oligomerisierung kann in der Flüssigphase, in einer Gasflüssigmischphase oder in der Gasphase durchgeführt werden. Bevorzugt wird die Oligomerisierung in der Flüssigphase durchgeführt.

Das erfindungsgemäße Verfahren wird zur Herstellung von Oligomeren aus einem Kohlenwasserstoffstrom (1), der überwiegend Butene enthält, verwendet. Dieser Kohenwasserstoffstrom (1) weist vorzugsweise mehr als 50 Massen-%, vorzugsweise zumindest 60 Massen-% und bevorzugt zumindest 70 Massen-% und besonders bevorzugt zumindest 80 Massen-% an Butenen auf.

Neben dem erfindungsgemäß erhaltenen Kohlenwasserstoffstrom (1) kann der in der Oligomerisierung eingesetzte Einsatzkohlenwasserstoffstrom einen oder mehrere weitere Kohlenwasserstoffströme, insbesondere C₄-Kohlenwasserstoffströme aufweisen. Der oder die zusätzlich eingesetzten Kohlenwasserstoffströme weisen vorzugsweise zumindest 50 Massen-%, bevorzugt von 60 bis 85 Massen-% an Butenen auf. Als zusätzliche Kohlenwasserstoffströme können beispielsweise solche eingesetzt werden, die bei der Aufarbeitung von C₄-Fraktionen eines FC-Crackers (Fluid Catalytic Cracker) erhalten werden, Kohlenwasserstoffströme, die bei der Aufarbeitung von C₄-Schnitten aus Steamcrackem erhalten werden und/oder Kohlenwasserstoffströme, die ganz oder teilweise in einem Verfahren zur Oligomerisierung durch Abtrennung vom Produkt der Oligomerisierung als Ströme erhalten wurden und die in der Oligomerisierung nicht umgesetzte C₄-Kohlenwasserstoffe aufweisen.

In dem erfindungsgemäßen Verfahren wird in Schritt a) ein gesättigte und ungesättigte C₄-Kohlenwasserstoffe enthaltender Strom (2) eingesetzt. Dieser Strom weist als gesättigte Kohlenwasserstoffe vorzugsweise n-Butan und optional Isobutan und als ungesättigte Kohlenwasserstoffe 1-Buten, cis-2-Buten und/oder trans-2-Buten auf. Neben den zu trennenden Butanen und Butenen können weitere Kohlenwasserstoffe in dem Kohlenwasserstoffstrom (2) vorliegen, die eine Anzahl Kohlenstoffatome größer oder kleiner als die zu trennenden C₄-Kohlenwasserstoffe aufweisen. Vorzugsweise weist der Kohlenwasserstoffstrom (2) neben den zu trennenden C₄-Kohlenwasserstoffen solche Kohlenwasserstoffe auf, die maximal zehn, bevorzugt 5, 4, 3 oder 2 und ganz besonders bevorzugt 1 Kohlenstoffatom(e) mehr, oder 3, 2 oder 1 Kohlenstoffatome weniger, als die zu trennenden Kohlenwasserstoffe aufweisen. Bevorzugt werden in dem erfindungsgemäßen Verfahren in Schritt a) Kohlenwasserstoffströme (2) eingesetzt, die als gesättigte Kohlenwasserstoffe Butane, insbesondere n-Butan und/oder Isobutan und als ungesättigte Kohlenwasserstoffe 1-Buten, cis-2-Buten und/oder trans-2-Buten und gegebenenfalls Isobuten und optional 1,3-Butadien aufweisen. Besonders bevorzugt können in dem erfindungsgemäßen Verfahren als Kohlenwasserstoffströmen (2) solche eingesetzt werden, die nahezu vollständig aus n-Butan, cis-Buten, trans-Buten und 1-Buten bestehen. Weitere Komponenten wie Isobutan, Isobuten, C₁-C₃- und C₅+-Kohlenwasserstoffe sind darin vorzugsweise mit unter 5 Massen-%, besonders mit unter 2 Massen-% Anteil enthalten. Die in Schritt a) einzusetzenden Kohlenwasserstoffströme (2) weisen vorzugsweise zumindest 50 Massen-%, bevorzugt zumindest 75 Massen-% und besonders bevorzugt 95 Massen-% an Butanen und Butenen auf. Besonders bevorzugt wird in Stufe a) ein Kohlenwasserstoffstrom (2) eingesetzt, der von 5 bis 75 Massen-%, vorzugsweise von 10 bis 65 Massen-% und besonders bevorzugt von 15 bis 50 Massen-% an Butenen aufweist.

Es kann vorteilhaft sein, wenn in den eingesetzten Kohlenwasserstoffströmen C₅-Kohlenwasserstoffe und höhere Kohlenwasserstoffe (C₅₊-Kohlenwasserstoffe) enthalten sind, diese vor dem Eintritt in die Stufe a), aus der Kopffraktion der Stufe a) und/oder aus der Kopffraktion der Stufe b), gegebenenfalls nach durchlaufen weiterer Prozessschritte, vollständig oder teilweise destillativ abzutrennen.

Geeignete Einsatzstoffe für den Einsatz in Schritt a) des erfindungsgemäßen Verfahrens sind beispielsweise Kohlenwasserstoffströme, die bei der Aufarbeitung von C₄-Fraktionen eines FC-Crackers erhalten werden, Kohlenwasserstoffströme, die bei der Aufarbeitung von C₄-Schnitten aus Steamcrackem erhalten werden und/oder Kohlenwasserstoffströme, die ganz oder teilweise in einem Verfahren zur Oligomerisierung durch Abtrennung vom Produkt der Oligomerisierung als Ströme erhalten wurden und die in der Oligomerisierung nicht umgesetzte C₄-Kohlenwasserstoffe aufweisen.

Die C₄-Fraktionen aus FC-Crackem enthalten in der Regel 20 bis 70 Massen-% an Butanen und 30 bis 80 Massen-% an Butenen. Ein gegebenenfalls vorhandener Rest kann aus sonstigen C₃-C₅ Kohlenwasserstoffen bestehen. Eine typische Zusammensetzung eines C₄-Schnittes aus einem FC-Cracker weist die folgende Zusammensetzung auf:

| | |
|---|---|
| Propan | 0,3 Massen-% |
| Propen | 1,2 Massen -% |
| n-Butan | 12 Massen -% |
| i-Butan | 30 Massen -% |
| 1-Buten | 14 Massen -% |
| i-Buten | 10 Massen -% |
| trans-2-Buten | 16 Massen -% |
| cis-2-Buten | 14 Massen -% |
| 1,3-Butadien | 0,5 Massen -% |
| C₅-Kohlenwasserstoffe | 2 Massen -% |

Das in der C₄-Fraktion von FC-Crackem enthaltene Butadien und gegebenenfalls vorhandene acetylenisch ungesättigte Verbindungen sollte vor Einsatz im erfindungsgemäßen Verfahren entfernt werden. Bevorzugt werden sie aus der C₄-Fraktion durch eine selektive Hydrierung, z. B. gemäß EP-PS 0 081 041 und DE-PS 15 68 542 entfernt, besonders bevorzugt durch eine selektive Hydrierung bis auf einen Restgehalt von kleiner 5 Massen-ppm. Das enthaltene Isobuten kann optional vor dem Einsatz im erfindungsgemäßen Verfahren entfernt werden, beispielsweise durch Veretherung mit Alkoholen, insbesondere mit Methanol oder Ethanol, oder durch andere selektive chemische Umsetzungen, beispielsweise mit Wasser zu tert.-Butanol.

Die nach selektiver Hydrierung der mehrfach ungesättigten Kohlenwasserstoffe, Abtrennung von Isobuten und Isobutan erhaltene Mischung von C₄-Kohlenwasserstoffen, die hauptsächlich 1-Buten, 2-Buten und n-Butan enthält, ist ein bevorzugter als Kohlenwasserstoffstrom (2) eingesetzter Einsatzstoff für das erfindungsgemäße Verfahren.

Weitere bevorzugte Kohlenwasserstoffströme, die als Kohlenwasserstoffstrom (2) für den Einsatz in Schritt a) des erfindungsgemäßen Verfahrens besonders geeignet sind, sind C₄-Kohlenwasserstoffströme, die bei der Aufarbeitung von C₄-Schnitten aus Steamcrackem erhalten werden. Die gegebenenfalls enthaltenen mehrfach ungesättigten Verbindungen werden hieraus abgetrennt oder selektiv hydriert. Aus der verbleibenden Mischung kann das Isobuten durch selektive chemische Umsetzungen, beispielsweise zu tert.-Butylethem wie Methyl-tert.-butylether oder Ethyl-tert.-butylether oder zu tert.-Butanol, abgetrennt werden. Die auf diese Weise erhaltene Mischung von C₄-Kohlenwasserstoffen enthält hauptsächlich 1-Buten, 2-Butene, n-Butan und Isobutan und wird technisch oftmals als Raffinat II bezeichnet. Aus dem Raffinat II können Isobutan und 1-Buten destillativ, vollständig oder teilweise, abgetrennt werden. Die zurückbleibende Mischung, Raffinat III, besteht üblicherweise hauptsächlich aus n-Butenen und n-Butan und ist ein besonders bevorzugtes Einsatzstoffgemisch für den Einsatz als Kohlenwasserstoffstrom (2) in Schritt a) des erfindungsgemäßen Verfahrens.

Ein weiteres bevorzugtes Einsatzstoffgemisch für den Einsatz als Kohlenwasserstoffstrom (2) in Schritt a) des erfindungsgemäßen Verfahrens sind C₄-Kohlenwasserstoffströme, die aus einem Verfahren zur Oligomerisierung von Butenen nach Abtrennung vom Produkt der Oligomerisierung als Strom erhalten wurden, und die die in der Oligomerisierung nicht umgesetzten C₄-Kohlenwasserstoffe enthalten.

In großtechnisch betriebenen Oligomerisierungen von n-Butenen aus Mischungen von n-Butenen und Butan werden Di-, Tri- und höhere Oligomere erhalten. Da ein vollständiger Umsatz der Butene ökonomisch üblicherweise nicht sinnvoll ist, fällt eine Mischung von n-Butenen und Butan an, die rein destillativ nicht wirtschaftlich aufgetrennt werden kann. Diese Mischung stellt ein weiteres bevorzugtes Einsatzstoffgemisch für den Einsatz als Kohlenwasserstoffstrom (2) in Schritt a) des erfindungsgemäßen Verfahrens dar. Über das erfindungsgemäße Verfahren können die noch enthaltenen Butene ebenfalls in einer Oligomerisierung genutzt werden. Zusätzlich wird eine Butan reiche Fraktion erhalten, aus der man beispielsweise hochreines Butan erhalten kann, das im Wesentlichen frei von geruchsverändernden Stoffen ist. Die Oligomerisierung, aus der die C₄-Kohlenwasserstoffströme erhalten werden, die als Kohlenwasserstoffstrom (2) in Stufe a) eingesetzt werden, kann vollständig oder teilweise identisch mit der Oligomerisierung sein, in der die im Kohlenwasserstoffstrom (1) enthaltenen Butene umgesetzt werden.

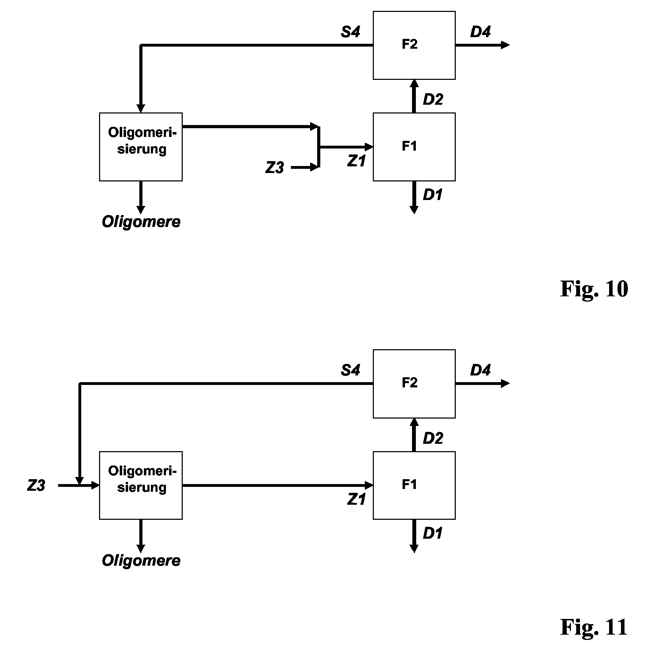

In einer ganz besonders bevorzugten Ausführungsart des erfindungsgemäßen Verfahrens kann in Stufe a) als Kohlenwasserstoffstrom (2) ein Gemisch eingesetzt werden, das ganz oder teilweise bei der erfindungsgemäßen Oligomerisierung nach Abtrennung vom Produkt der Oligomerisierung als Strom erhalten wurde, der in der Oligomerisierung nicht umgesetzte C₄-Kohlenwasserstoffe aufweist. Eine solche bevorzugte Ausführungsart des erfindungsgemäßen Verfahrens ist in den Figuren Fig. 10 bis Fig. 13 schematisch dargestellt.

Wie auch aus den angegebenen Figuren ersichtlich ist, kann das erfindungsgemäße Verfahren besonders vorteilhaft genutzt werden, wenn in der Oligomerisierung nur ein Teil der Butene umgesetzt, vorzugsweise nur 60 bis 90 % umgesetzt wird und die nicht umgesetzten Butene, nach Abtrennung der Oligomerisierungsprodukte, in Stufe a) zumindest teilweise wieder eingesetzt werden.

Für die Zuführung eines Kohlenwasserstoffstroms (2) zur Stufe a) des erfindungsgemäßen Verfahren ergeben sich dabei drei bevorzugte Möglichkeiten:
1) In Stufe a) wird in diesem Fall ein Kohlenwasserstoffstrom (2) eingesetzt, der eine Mischung aus einem Kohlenwasserstoffstrom, der aus einer Oligomerisierung nach Abtrennung von Oligomeren erhalten wurde und der Butene aufweist, und einem externen Kohlenwasserstoffstrom ist. Diese Option ist besonders bei externen Kohlenwasserstoffströmen (nicht aus dem erfindungsgemäßen Verfahren stammenden Strömen) mit geringen Buten-Gehalten, beispielsweise aus der Aufarbeitung von FC-Crackem, bevorzugt.
2) In Stufe a) werden ausschließlich die aus der Oligomerisierung nach Abtrennung der Oligomeren stammenden Butene eingesetzt. Die Zuführung der externen Kohlenwasserstoffströme in die Oligomerisierung kann dabei zusammen mit dem aus der Stufe d) erhaltenen Kohlenwasserstoffstrom (1) oder an anderer Stelle der Oligomerisierung, bei mehrstufigen Oligomerisierungsprozessen beispielsweise in unterschiedliche Stufen, erfolgen.
3) Der externe Kohlenwasserstoffstrom wird schon zusammen mit der Kopffraktion (5) in Stufe d) des erfindungsgemäßen Verfahrens geführt, wo er gemeinsam mit der Kopffraktion (5) getrocknet wird. In einer speziellen Ausführungsform wird diese Trocknung wie nachfolgend beschrieben in einer Trocknungskolonne durchgeführt, bei der am Kopf Wasser und ggf. eine Kohlenwasserstofffraktion (beispielsweise Isobutan, 1-Buten) abgenommen wird.

Es kann vorteilhaft sein, wenn die Sumpffraktion aus Stufe b) zumindest teilweise als Extraktionsmittel in die Stufe a) eingespeist wird. Bevorzugt wird die Sumpffraktion aus Stufe b) vollständig oder nahezu vollständig als Extraktionsmittel in die Stufe a) zurückgeführt. Damit im Kreislaufstrom des Extraktionsmittels sich anreichernde Fremdstoffe (beispielsweise Zersetzungsprodukte des Extraktionsmittels) aus dem Prozess entfernt werden können, kann es vorteilhaft sein, wenn ein Teilstrom kontinuierlich oder chargenweise ausgeschleust und entweder regeneriert oder durch frisches Extraktionsmittel ersetzt wird. Um Schwankungen im Prozess auszugleichen, kann es vorteilhaft sein, einen Zwischen- oder Lagerbehälter für das Extraktionsmittel vorzusehen.

In einer bevorzugten Ausführungsform des erfindungsgemäßen Verfahrens wird die Sumpffraktion aus Stufe b) zumindest teilweise als Extraktionsmittel in die Stufe a) eingespeist und die in der Sumpffraktion enthaltene Wärmeenergie wird in einem Wärmetauscher genutzt, um den Zulauf zur Destillationskolonne der Stufe b) zu erwärmen.

Vorzugsweise wird in dem erfindungsgemäßen Verfahren die Destillation gemäß Stufe a) oder Stufe b) in einer Apparatur durchgeführt, die einen Dekanter aufweist, und in diesem Dekanter die kondensierte Kopffraktion aus der Destillation in einen unpolaren Strom, der Kohlenwasserstoffe aufweist, und einen polaren Strom, der das Extraktionsmittel aufweist, getrennt. Zu diesem Zweck werden die als Brüden am Kolonnenkopf erhaltenen Strome zunächst so behandelt, dass der Stoffstrom oder Teile davon als flüssige Phase vorhanden sind. Das Behandeln kann z. B. durch Abkühlung mit oder ohne vorheriger Verdichtung erfolgen, so dass zumindest teilweise eine flüssige Phase erhalten wird.

In dem erfindungsgemäßen Verfahren kann es vorteilhaft sein, wenn sowohl die extraktive Destillation in Stufe a), als auch die destillative Trennung in Stufe b), in einer Apparatur durchgeführt wird, die einen Dekanter aufweist. Sind mehrere Apparaturen zur Durchführung von mehreren Stufen a) und/oder b) vorhanden, so können alle oder aber auch nur einige der Apparaturen mit einem Dekanter ausgerüstet sein.

Der aus dem Dekanter oder den Dekantem erhaltene polare und gegebenenfalls wässrige Strom kann zumindest teilweise direkt oder nach einer Aufarbeitung in den Prozess zurückgeführt werden. Die Rückführung kann in Stufe a), hier bevorzugt in den Zulaufstrom des Extraktionsmittels, oder in Stufe b), oder in einen meist vorhandenen Vorratsbehälter für das Extraktionsmittel, erfolgen. Sind mehrere Stufen a) und/oder b) vorhanden, so kann es vorteilhaft sein, den polaren Strom aus dem Dekanter oder den Dekantem in die erste Stufe der Stufe a) zurückzuführen. Vorzugsweise wird der aus dem Dekanter oder den Dekantem erhaltene polare, wässrige Strom zumindest teilweise dem Vorratsbehälter für das polare Extraktionsmittel zugeführt. Auf diese Weise kann auf zusätzliche Pumpen verzichtet werden.

Die Extraktionsdestillation der Stufe a) wird bevorzugt bei einem Druck von 0,2 bis 1,5 MPa und einer Temperatur von 40 bis 100 °C betrieben. Üblicherweise werden solche Kolonnen im Gegenstrom betrieben, d. h. das Extraktionsmittel wird oberhalb des zu extrahierenden Stroms in die Kolonne eingeführt. Bevorzugt wird der zu extrahierende Strom (der zu behandelnde C₄₋Strom) in das mittlere Drittel der Kolonne eingeführt. Im vorliegenden Fall wird der zu extrahierende C₄-Kohlenwasserstoffstrom bevorzugt vor der Kolonne verdampft und als Gasstrom im Massenverhältnis von 15: 1 bis 6:1, bevorzugt 12: 1 bis 6: 1 (Gas : Flüssigkeit) mit dem polaren Extraktionsmittel in Kontakt gebracht. Zweckmäßiger Weise wird die Kolonne mit Einbauten oder Füllkörpern zum Aufbau einer möglichst großen Austauschfläche ausgestattet. Als Einbauten für die Extraktivdestillationskolonne haben sich insbesondere Füllkörper, Glocken- oder Ventilböden bewährt. Eine bevorzugt eingesetzte Extraktionskolonne weist von 10 bis 50, vorzugsweise 15 bis 25 theoretische Trennstufen. Vorzugsweise wird die erfindungsgemäß in Stufe a) eingesetzte Extraktionskolonne mit einer Berieselungsdichte von 10 bis 100, vorzugsweise 40 bis 60 m³/(m² * h) gefahren.

Oberhalb der Zuführung des Extraktionsmittels wird in der Kolonne vorzugsweise ein Destillationsabschnitt vorgesehen, mit dem der Anteil an Extraktionsmittel im Kopfprodukt der Kolonne verringert wird. Bevorzugt erstreckt sich dieser Abschnitt bis zum Kolonnenkopf. Auch hier werden zweckmäßigerweise Einbauten, Packungen oder Füllkörper in den Kolonnenabschnitt eingebracht, die gleich oder verschieden zu denen im Kolonnenabschnitt unterhalb des Extraktionsmittelzulaufs sein können. Es kann zudem sinnvoll sein, diesen Kolonnenabschnitt in einem geringeren Kolonnendurchmesser als den unteren Kolonnenteil auszuführen, beispielsweise um die Berieselungsdichte zu optimieren.

Am Kopf der Kolonne kann gasförmig das hauptsächlich Butane aufweisende Gemisch abgezogen und einer weiteren Verwendung zugeführt werden. Vorzugsweise wird das Kopfprodukt der Extraktionsdestillation gemäß Stufe a) aber z. B. durch Abkühlung mit oder ohne vorherige Verdichtung kondensiert und in einen Dekanter überführt und dort in einen polaren und einen unpolaren Strom getrennt. Der polare Strom, der Reste des Extraktionsmittels aufweist, kann beispielsweise in den Zulauf der Kolonne zurückgeführt oder aber anderweitig verwertet werden.

Der unpolare Strom, der am Kopf der Kolonne der Stufe a) erhalten wird, enthält weniger als 40 Massen-% an ungesättigten C₄-Kohlenwasserstoffen, bevorzugt weniger als 25 Massen-%, besonders bevorzugt weniger als 15 Massen-%. Er kann ganz oder teilweise einer weiteren Aufarbeitung, z. B. einer destillativen Aufarbeitung zugeführt werden. Ein Teil kann als Rücklauf in die Kolonne der Stufe a) zurückgeführt werden. Bevorzugt wird ein Rücklauf vorgesehen, wobei vorzugsweise ein Rücklaufverhältnis von 0,05 bis 17 [kg/kg], bevorzugt 0,5 bis 4 [kg/kg], definiert als das Verhältnis der in die Kolonne zurückgeführten Menge zur abgeführten Menge des unpolaren Stroms, eingestellt wird.

Die Aufarbeitung des unpolaren, Butane aufweisenden Stroms kann z. B. dadurch erfolgen, dass dieser z. B. in einer Waschkolonne mit Wasser oder einer wässrigen Lösung gewaschen wird (Stufe e)). Der mit Wasser gesättigte unpolare Strom der bei dieser Wäsche gemäß Stufe e) erhalten wird oder aber auch der unpolare Strom, der am Kopf der Kolonne der Stufe a) erhalten wird, kann dann in einer weiteren Destillationskolonne in einen nahezu wasserfreien unpolaren Strom aufgearbeitet werden (Stufe f)). Zu diesem Zweck kann diese Destillationskolonne ebenfalls wieder einen Dekanter aufweisen, in welchem das kondensierte Kopfprodukt in eine organische Phase, die zu 50 bis 100 Massen-% in die Kolonne zurückgefahren wird, und eine wässrige Phase, die ausgeschleust wird, aufgetrennt wird. Der nicht in die Kolonne zurückgefahrene Strom, der Leichtsieder wie beispielsweise Isobutan enthält, kann anderweitig weiterverwendet werden. Das in dieser Destillationskolonne erhaltene Sumpfprodukt weist einen Wassergehalt von kleiner 50 wppm (Massen-ppm), besonders bevorzugt kleiner 5 wppm auf. Bei Ausschleusung eines Teils der organischen Phase des Kopfproduktes wird außerdem ein Sumpfprodukt erhalten, welches im Vergleich zum in die Stufe f) eingeführten Ausgangsprodukt an Leichtsiedern, beispielsweise an Isobutan, abgereichert ist. Die Destillationskolonne der Stufe f) wird vorzugsweise mit einer Kopftemperatur von 40 bis 60 °C, besonders bevorzugt mit einer Kopftemperatur von 45 bis 55 °C betrieben.

Durch die Wäsche und/oder anschließende Abtrennung des Wassers aus dem Kopfprodukt der Stufe a) in diesen optionalen Stufen e) und/oder f) können polare Verunreinigungen, die insbesondere den Geruch von gesättigten Kohlenwasserstoffen, wie z. B. Butanen beeinflussen, weitestgehend abgetrennt werden.

Eine weitere Möglichkeit der Aufarbeitung des unpolaren Stroms, der am Kopf der Kolonne der Stufe a) erhalten wird, oder eines unpolaren Stroms, wie er aus den optionalen Stufen e) und/oder f) erhalten wird, vorzugsweise des aus der Stufe f) erhaltenen butanreichen Sumpfproduktes, kann aus einer Entfernung von evtl. noch vorhandenen ungesättigten Kohlenwasserstoffen in einer optionalen Hydrierstufe g) bestehen. In dieser Stufe können die im unpolaren Strom, der am Kopf der Kolonne der Stufe a) oder aus den optionalen Stufen e) und/oder f) erhalten wird, gegebenenfalls noch enthaltenen Olefine zu Alkanen umgesetzt werden. Die Hydrierstufe g) kann im einfachsten Fall aus einem Hydrierreaktor bestehen, in dem die ungesättigten Olefme gemäß dem Stand der Technik zu Alkanen umgesetzt werden. Optional kann die Hydrierstufe noch eine oder mehrere Destillationskolonnen aufweisen, in denen das aus dem Hydrierreaktor erhaltene Produkt weiter destillativ getrennt wird. Z. B. können in einer solchen Destillationskolonne C₁-C₃-Kohlenwasserstoffe und/oder Kohlenwasserstoffe mit 5 oder mehr Kohlenstoffatomen abgetrennt werden. Sind in den Butanen sowohl n-Butan als auch i-Butan enthalten, können diese destillativ auf die reinen Stoffe aufgetrennt werden.

Um Spuren von weiteren Verunreinigungen zu entfernen kann es vorteilhaft sein, den unpolaren Strom, der am Kopf der Kolonne der Stufe a) erhalten wird, oder einen unpolaren Strom, wie er aus einer oder mehreren der optionalen Stufen e) und/oder f) und/oder g) erhalten wird, zusätzlich oder ersatzweise einer Nachreinigung (optionale Stufe h)) zuzuführen, in welcher eine Behandlung mit einem oder mehreren Adsorbern, z. B. in Adsorberbetten, erfolgt. Gebräuchliche Adsorbentien sind z. B. Aktivkohle und Molekularsiebe.

Besonders bevorzugt wird der unpolare Strom, der am Kopf der Kolonne der Stufe a) erhalten wird, zunächst in einer Stufe e) gewaschen und anschließend in einer Stufe f) getrocknet. Der getrocknete unpolare Strom aus f) wird anschließend einer Hydrierungsstufe g) zugeführt und danach noch in Stufe h) mit einem Adsorber behandelt. Auf diese Weise können die Butane aufweisenden unpolaren Ströme, die am Kopf der Kolonne der Stufe a) erhalten werden, zu hochreinem n-Butan aufgereinigt werden.

Eine alternative, bevorzugte Verfahrensvariante kann dadurch realisiert werden, dass eine Hydrierstufe i) zwischen den Verfahrensstufen e) und f) durchlaufen wird. Bei dieser Ausführungsvariante des erfindungsgemäßen Verfahrens wird das Produkt aus der Wäsche gemäß Stufe e) zunächst in einen Hydrierreaktor gefahren, in dem nach bekannten Verfahren ungesättigte Kohlenwasserstoffe zu gesättigten Kohlenwasserstoffe hydriert werden. Das Produkt aus dieser Hydrierung wird dann direkt in den Destillationsschritt gemäß Stufe f) gefahren, in welcher als Sumpfprodukt der gesättigte Kohlenwasserstoff erhalten wird. Ein Vorteil dieser Verschaltung kann darin liegen, dass am Kopf der Kolonne kein vollständiger Rücklauf gefahren wird, sondern C₁-C₃-Kohlenwasserstoffe und Isobutan als Teilstrom einfach abgetrennt werden können.

Im Sumpf der Kolonne aus Stufe a) reichem sich das Extraktionsmittel und insbesondere die ungesättigten Kohlenwasserstoffe aus dem aufzutrennenden Gemisch an. Der Sumpf der Kolonne wird bevorzugt extern beheizt.

Die aus Stufe a) erhaltene Sumpffraktion wird der Destillationskolonne der Stufe b) zugeleitet. Vorzugsweise wird die Sumpffraktion vor dem Einleiten in die Destillationskolonne der Stufe b) durch Wärmetausch mit Dampf oder einem anderen Heizmedium, vorzugsweise durch indirekten Wärmetausch mit der Sumpffraktion der Stufe b), erwärmt. Sollte bei der Verwendung der Sumpffraktion der Stufe b) als Heizmedium die in der Sumpffraktion enthaltene Wärmeenergie nicht ausreichend sein, um die gewünschte Wärmeenergie zu übertragen, können weitere Wärmetauscher vorgesehen werden, in denen der der Stufe b) zugeleitete Strom mittels anderer Heizmedien, wie beispielsweise Dampf oder andere Prozessströme, aufgeheizt werden kann. Der Zulauf zur Kolonne der Stufe b) erfolgt bevorzugt in der oberen Hälfte, besonders bevorzugt im oberen Drittel der Kolonne der Stufe b).

In einer bevorzugten Ausführungsform des erfindungsgemäßen Verfahrens kann der Zulauf zur Destillationskolonne gemäß Stufe b) unter einem Druck erwärmt werden, der höher ist als der Druck in der Destillationskolonne b). Der Zulauf wird bei dieser Ausführungsform nach dem Erwärmen in die Destillationskolonne gemäß Stufe b) entspannt.

In einer weiteren bevorzugten Ausführungsform des erfindungsgemäßen Verfahrens kann der Zulauf zur Destillationskolonne gemäß Stufe b) unter einem Druck erwärmt werden, der dem Druck in der Destillationskolonne b) entspricht. Vorzugsweise wird der Zulauf dabei so weit erwärmt, dass er zumindest teilweise verdampft. Es kann dabei vorteilhaft sein, den Zulauf zur Destillationskolonne gemäß Stufe b) vor Eintritt in die Kolonne in eine Dampfphase und eine Flüssigphase aufzutrennen, und diese Phasen einzeln auf unterschiedlichen oder gleichen, vorzugsweise unterschiedlichen Böden der Destillationskolonne des Schrittes b) einzuspeisen. Besonders bevorzugt erfolgt die Einspeisung der dampfförmigen Phase 1 bis 5 theoretische Trennstufen oberhalb der Einspeisung der flüssigen Phase. Ein bevorzugte Ausführungsform einer Apparatur zur Erwärmung des Zulaufs ist in der Fachliteratur beispielsweise als Kettle-Verdampfer bekannt.

Die Stufe b) wird vorzugsweise bei einem Druck von 0,1 bis 1 MPa, vorzugsweise 0,3 bis 0,5 MPa und bei einer Temperatur von 120 bis 230 °C vorzugsweise 125 bis 190 °C betrieben. Der Kolonnensumpf kann in die Extraktionsdestillation gemäß Stufe a) zurückgeführt werden. Die Beheizung der Kolonne erfolgt durch einen Sumpfverdampfer, der bevorzugt mit Dampf betrieben wird.

Am Kopf der Destillationskolonne gemäß Stufe b) werden die Brüden zumindest teilweise kondensiert. Das Kondensieren kann durch Abkühlen mit oder ohne vorherigem Verdichten erfolgen. Das kondensierte Kopfprodukt wird anschließend bevorzugt in einen unpolaren und einen polaren Strom getrennt. Zu diesem Zweck wird das kondensierte Kopfprodukt bevorzugt in einen Dekanter geleitet. Der polare Strom kann ganz oder teilweise in den Prozess zurückgeführt werden oder aber verworfen oder aufgearbeitet werden. Der unpolare Strom kann zum Teil in die Stufe b) als Rücklauf zurückgeführt werden, der Rest wird ganz oder teilweise der Stufe c) des erfindungsgemäßen Verfahrens zugeführt. Bevorzugt wird ein Rücklauf vorgesehen, wobei vorzugsweise ein Rücklaufverhältnis von 0,05 bis 17 [kg/kg], bevorzugt 0,5 bis 4 [kg/kg], defmiert als das Verhältnis der in die Kolonne zurückgeführten Menge zur abgeführten Menge des unpolaren Stroms, eingestellt wird.

Ein erfindungsgemäßer Dekanter kann in der jeweiligen Apparatur zur Durchführung der Stufen a) und/oder b) integriert sein oder als eigenständige Apparatur vorhanden sein. Bevorzugt ist der Dekanter in der jeweiligen Apparatur zur Durchführung der Stufen a) und/oder b) in dem Kopfkondensator oder in der Destillatvorlage (den Destillatsvorlagenbehälter) der Kolonne integriert. Im erfindungsgemäßen Verfahren verwendbare Apparaturen oder Dekanter sind standardmäßig bei Apparatebauern zu beziehen. Eine Literaturübersicht und Auslegungsregeln findet man in M. Henschke; Dimensionierung liegender Flüssig-flüssig-Abscheider anhand diskontinuierlicher Absetzversuche; Fortschritt-Berichte VDI, Reihe 3: Verfahrenstechnik, Nr. 379, Düsseldorf 1995.

In einer besonderen Ausführungsform des erfindungsgemäßen Verfahrens kann es vorteilhaft sein, wenn ein Teil der unkondensierten Kopffraktion der Stufe b) in die Extraktionsstufe a) zurückgeführt wird. Vorzugsweise werden zwischen 0 und 51 Massen-% des unkondensierten Kopfstroms der Stufe b) in die Stufe a) zurückgeführt. Wird die Stufe a) bei einem höheren Druck als die Stufe b) betrieben, wird der in die Stufe a) zurückgeführte Teil der Kopffraktion der Stufe b) auf den Betriebsdruck der Stufe a), z. B. mit einer Verdichterstufe, verdichtet. Die Rückführung eines Teils der Kopffraktion aus Stufe b) in die Stufe a) erfolgt vorzugsweise in das untere Viertel, besonders bevorzugt in den Sumpf der Kolonne von Stufe a).

Als weitere besondere Ausführungsform kann, als Alternative zur Rückführung von Brüden in die Stufe a), ein Teil des unpolaren, kondensierten Stroms der Stufe b) in die Stufe a) zurückgeführt werden.

Bevorzugt wird die Kolonne der Stufe b) bei einem Druck größer 0,3 MPa betrieben, so dass die Kondensation durch Abkühlen mit Rückkühlwasser oder Flusswasser erfolgen kann. Liegt der Kolonnendruck niedriger, kann eine Kondensation nach Verdichtung der Brüden oder durch Einsatz von Kühlmedien tieferer Temperatur (beispielsweise Kühlsole) erfolgen.

Der unpolare Strom aus dem Kopfprodukt der Stufe b) enthält neben ungesättigten Kohlenwasserstoffen vorzugsweise weniger als 40 Massen-% an gesättigten Kohlenwasserstoffen, bevorzugt weniger als 25 Massen-% gesättigte Kohlenwasserstoffe, besonders bevorzugt weniger als 20 Massen-% gesättigte Kohlenwasserstoffe.

Die bei der Kondensation der Brüden in Stufe b) anfallende Kopffraktion (5), insbesondere der nicht zurückgeführte, unpolare Teil der Kopffraktion wird in Stufe c) mit Wasser oder einer wässrigen Lösung gewaschen. Eine solche flüssig-flüssig Extraktion ist dem Fachmann aus seinem Allgemeinwissen und dem Stand der Technik bekannt. Die Wäsche gemäß Stufe c) kann ein oder mehrstufig durchgeführt werden. Vorzugsweise wird die Wäsche mehrstufig durchgeführt, insbesondere wenn zur Wäsche Mixer-Settler-Einheiten eingesetzt werden. Die Wäsche kann aber auch in einer oder mehreren Extraktionskolonnen durchgeführt werden.

Bevorzugt wird die Extraktion in einer Extraktionskolonne durchgeführt. Die Anzahl der in der Extraktionskolonne realisierten theoretischen Stufen beträgt bevorzugt 1 bis 50, besonders bevorzugt 5 bis 25, besonders bevorzugt 10 bis 15 Stufen. Das Verhältnis von wässriger zu organischer Phase beträgt 2:1 bis 1:25, bevorzugt 1:5 bis 1: 15.

Der nach der Wäsche erhaltene mit Wasser gesättigte unpolare Strom wird in einer Stufe d) unter Erhalt eines weitgehend wasserfreien butenhaltigen Kohlenwasserstoffstroms (1) getrocknet. Für die Trocknung können die gängigen Standardverfahren wie beispielweise , Destillationen, Druckwechseladsorptionen oder Membrantrocknungen verwendet werden. Bevorzugt erfolgt die Trocknung des unpolaren Stroms durch Überführung des Stroms in eine Destillationskolonne (Trocknungskolonne), in welcher als Sumpfprodukt ein getrockneter, weitgehend wasserfreier, Butene aufweisender, unpolarer Strom erhalten wird.

Der Wassergehalt im Sumpf der Trocknungskolonne beträgt vorzugsweise kleiner 50 ppm, bevorzugt kleiner 10 ppm, besonders bevorzugt kleiner 5 ppm (Massen-ppm). Die Destillationskolonne wird vorzugsweise mit einer Kopftemperatur von 40 bis 60 °C, besonders bevorzugt mit einer Kopftemperatur von 45 bis 55 °C betrieben. Am Kopf der Destillationskolonne wird das gasförmige Kopfprodukt kondensiert und kann in einen Dekanter geleitet werden. In dem Dekanter kann das kondensierte Kopfprodukt in einen polaren Strom und einen unpolaren Strom getrennt werden. Der unpolare Strom kann teilweise als Rücklauf in die Kolonne zurückgeführt werden. Der polare Strom, welcher insbesondere das Wasser aus der Wäsche enthält, kann verworfen oder in die Wasserwäsche zurückgeführt werden. Der nicht zurückgeführte Teil des unpolaren Stroms kann anschließend als Kohlenwasserstoffstrom (1) der erfindungsgemäßen Oligomerisierung zugeführt werden.

Es kann vorteilhaft sein, wenn der butenhaltige Kohlenwasserstoffstrom (1) vor der Verwendung als Einsatzstoff in der Oligomerisierung durch in Kontaktbringen mit einem Adsorber nachgereinigt wird. Eine solche Nachreinigung an einem Molekularsieb als Adsorber wird z. B. in EP 0 395 857 beschrieben.

Als besonders vorteilhaft hat es sich erwiesen, wenn der Extraktionsmittel-Strom (4) in die Stufe a) zurückgeführt wird und die in (4) enthaltene Wärmeenergie durch indirekten Wärmetausch mit weiteren Prozessströmen weiter genutzt wird. Es kann vorteilhaft sein, die in der Sumpffraktion der Stufe b) nach Wärmetausch mit dem Zustrom zur Stufe b) enthaltene Wärmeenergie zumindest teilweise, bevorzugt vollständig zum Beheizen des Sumpfes der Stufe a), der Sümpfe der gegebenenfalls vorhandenen Stufe f) und/oder zur Verdampfung des Kohlenwasserstoff-Zulaufstroms zur Stufe a) und/oder in Stufe d), so diese eine Trocknungskolonne enthält, zur Beheizung selbiger einzusetzen. Auf diese Weise kann der Energieaufwand weiter reduziert werden.

Wie bereits erwähnt, kann das Einsatzkohlenwasserstoffgemisch (Kohlenwasserstoffstrom (2)) neben Kohlenwasserstoffen mit der gleichen Anzahl an Kohlenstoffatomen auch solche aufweisen, die mehr oder weniger Kohlenstoffatome aufweisen. So können in C₄-Kohlenwasserstoffgemischen insbesondere C₃- und/oder C₅-Kohlenwasserstoffe enthalten sein. Zur Abtrennung von C₅-Kohlenwasserstoffen (beispielsweise Isopentan, Neopentan) kann es vorteilhaft sein, eine optionale Stufe j) im erfindungsgemäßen Verfahren vorzusehen. Die Stufe j) wird beispielsweise dadurch realisiert, dass sie eine Destillationskolonne aufweist, in der die Kohlenwasserstoffe mit 5 oder mehr Kohlenstoffatomen (C₅₊-Kohlenwasserstoffe) als Sumpffraktion und die C₄-Kohlenwasserstoffe als Kopffraktion erhalten werden. Die Stufe j) kann vor der Stufe a) angeordnet sein, so dass aus dem Kohlenwasserstoffstrom (2) vor Eintritt in die Stufe a) bereits die C₅₊-Kohlenwasserstoffe im Wesentlichen abgetrennt werden. In diesem Fall wird das Kopfprodukt der Stufe j) bzw. ein Teil davon als Ausgangsprodukt in der Stufe a) eingesetzt. In einer Variante der Stufe j) wird eine Destillationskolonne eingesetzt, die eine ausreichende Trennleistung aufweist, um sowohl Kohlenwasserstoffe mit weniger als auch mit mehr als 4 Kohlenstoffatomen von den Kohlenwasserstoffen mit 4 Kohlenstoffatomen abzutrennen. Bei dieser Ausführungsvariante der Stufe j) wird das Produkt, welches in die Stufe a) geführt wird, als Mittelfraktion aus einem Seitenabzug der Kolonnen erhalten. Kohlenwasserstoffe mit mehr als 4 Kohlenstoffatomen werden als Sumpffraktion der Kolonne erhalten und Kohlenwasserstoffe mit weniger als 4 Kohlenstoffatomen werden als Kopffraktion erhalten.

Alternativ können die C₅₊-Kohlenwasserstoffe aber auch an anderer Stelle in der Aufarbeitung des Stroms der ungesättigten und/oder der gesättigten Kohlenwasserstoffe abgetrennt werden.

Beispielsweise kann der unpolare Teil der Kopffraktion von Stufe a) in die Stufe j) geleitet werden, wo wiederum die Kohlenwasserstoffe mit 5 oder mehr Kohlenstoffatomen als Sumpffraktion abgetrennt werden. Diese Aufarbeitung kann auch erst nach Durchlaufen der Stufe b), oder Durchlaufen der Stufen e) und f) usw. erfolgen. Die C₅₊-freien bzw. -ärmeren C₄-Kohlenwasserstoffe können entweder direkt verwendet werden oder einer weiteren Aufarbeitung z. B. gemäß Stufe g) zugeführt werden. Analog kann die Stufe j) auch in die Aufarbeitung des unpolaren Kopfproduktes der Stufe b) eingebunden werden.

Im erfindungsgemäßen Verfahren wird ein polares Extraktionsmittel eingesetzt. Bevorzugt wird ein Extraktionsmittel eingesetzt, welches ein Gemisch aus zumindest einem polaren organischen Extraktionsmittel und Wasser ist. Als polares organisches Extraktionsmittel kann z. B. eine oder mehrere Verbindungen, ausgewählt aus Dimethylformamid (DMF), N-Methylpyrrolidon (NMP), Acetonitril, Furfural, N-Formylmorpholin oder Dimethylacetamid, bevorzugt mit einem Anteil an Wasser (demineralisiertes Wasser) eingesetzt werden. Der Anteil des Wassers im Gemisch kann 1 bis 20 Massen-%, bevorzugt 3 bis 18 Massen-%, besonders bevorzugt 5 bis 12 Massen-%, insbesondere 8 bis 9 Massen-% betragen.

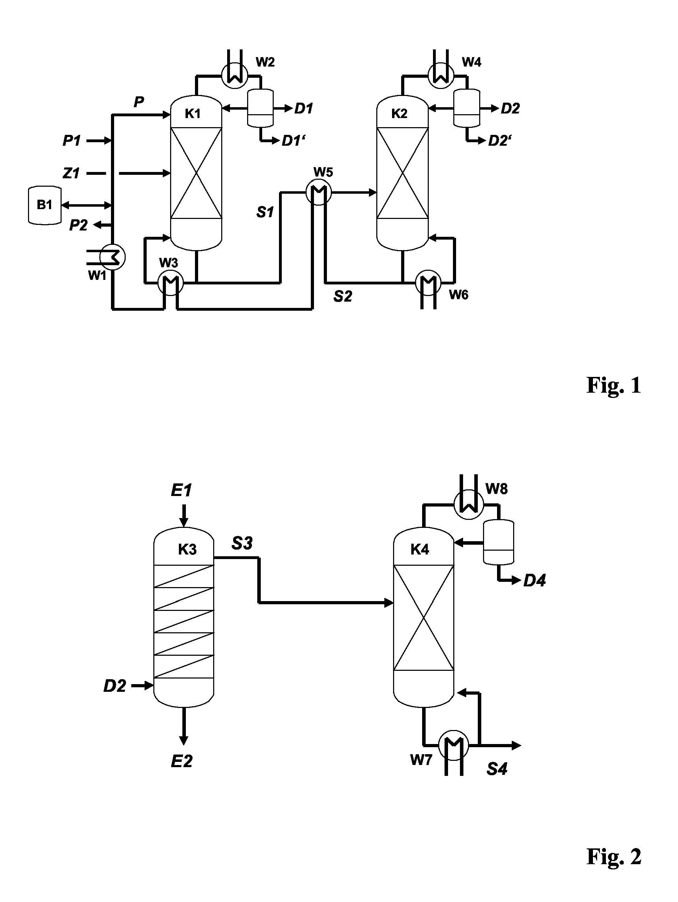

Die vorliegende Erfindung wird nachfolgend an Hand der in den Figuren **Fig. 1** bis **Fig. 13** wiedergegebenen Abbildungen und Schemata näher erläutert, ohne dass die Erfindung auf die dort beschriebenen Ausführungsformen beschränkt sein soll.

In **Fig. 1** sind die Extraktionskolonne K1, die Kolonne K2 (Ausgaser) und der Lösungsmittelkreislauf des Extraktionsmittels dargestellt (Stufen a) und b)). In den oberen Bereich der Kolonne K1 wird das Extraktionslösungsmittel P zugeführt. Der Zulauf der C₄-Kohlenwasserstoffe ZI erfolgt unterhalb der Zuführung des Extraktionsmittels und kann sowohl flüssig als auch gasförmig erfolgen. Am Kopf der Kolonne K1 wird im Kondensator W2 ein flüssiger Kopfstrom erhalten, der in einen Dekanter geführt wird. In diesem Dekanter wird der Kopfstrom in eine polaren Strom D1' und einen unpolaren Strom D1 aufgetrennt. Der unpolare Strom D1 enthält überwiegend Butane und wird teilweise als Rücklauf in die Kolonne K1 zurückgeführt. Der polare Strom D1' kann aufgearbeitet oder in den Prozess zurückgeführt werden.

Die Kolonne K1 wird über den Sumpfverdampfer W3 beheizt, in dem über indirekten Wärmetausch Wärme aus dem Extraktionsmittelkreislauf genutzt wird. Für den Fall, dass aus dem Extraktionsmittelkreislauf nicht genügend Energie genutzt werden kann, kann diese über einen zusätzlichen Sumpfverdampfer (nicht abgebildet) in die Kolonne eingebracht werden. Im Sumpf der Kolonne K1 fällt ein Strom S1, bestehend aus dem hauptsächlich mit Butenen beladenen Extraktionslösungsmittel, an. Oberhalb der Extraktionszone (oberhalb des Zulaufs des Extraktionsmittels) in der Kolonne K1 können optional Trennstufen vorgesehen werden, um den Anteil des Extraktionsmittels im Destillat abzusenken.

Der Strom S1 wird im Wärmetauscher W5 durch indirekten Wärmetausch mit dem Extraktionsmittelkreislauf erwärmt, bevorzugt teilweise verdampft und in die Kolonne K2 geleitet. Hier erfolgt die Ausgasung der gelösten C₄-Kohlenwasserstoffe, die am Kopf der Kolonne im Kondensator W4 kondensiert und in einen Dekanter geleitet werden. In diesem Dekanter wird das Kopfprodukt der Kolonne K2 in einen polaren Strom D2' und einen unpolaren Strom D2 aufgetrennt. Ein Teilstrom des unpolaren Stroms D2 wird als Rücklauf in die Kolonne zurückgeführt. Der polare Strom D2'kann aufgearbeitet oder in den Prozess zurückgeführt werden. Der Strom D2 wird in eine Wäsche überführt (siehe **Fig. 2).**

Im Sumpf der Kolonne K2 fällt das weitgehend von C₄-Kohlenwasserstoffen befreite Extraktionsmittel als Sumpfstrom S2 an. Die Beheizung der Kolonne erfolgt durch den Sumpfverdampfer W6, bevorzugt mit Dampf, beispielsweise 2,0 MPa-Dampf.

Das als Sumpfstrom S2 erhaltene Extraktionsmittel wird zur Ausnutzung der enthaltenen Wärme über die Wärmetauscher W5 (Vorwärmung Zulauf K2) und W3 (Sumpfverdampfer K1) über den Wärmetauscher W1, in dem das Extraktionsmittel auf die entsprechende Zulauftemperatur gebracht wird, in die Extraktionskolonne K1 zurückgeführt, womit sich der Extraktionsmittelkreislauf schließt. Über die Ströme P1 und P2 wird verbrauchtes Extraktionsmittel (P2) aus dem Extraktionsmittelkreislauf entnommen bzw. frisches (P1) hinzugefügt. In dem Behälter B1 können, falls notwenig, Schwankungen der Menge an Extraktionsmittel im Kreislauf abgepuffert werden.

Die Rückführung der Ströme D1' und/oder D2' kann an gleicher oder unterschiedlicher Stelle des Prozesses erfolgen. Bevorzugt ist die Rückführung in den Lösungsmittelkreislauf des Extraktionsmittels, beispielsweise in den Ausgleichsbehälter B 1 oder in den Zulauf des Extraktionsmittels zur K1.

Ein Möglichkeit zur Ausführung der Wäsche (Stufe c) des Destillats D2 ist in **Fig. 2** wiedergegeben. In einer Extraktionskolonne K3 wird das Destillat D2, bzw. der polare Teil des Destillats 2 mit einer Waschlösung von organischen Resten des Extraktionsmittels befreit. Als Waschlösungen E1 kommen Wasser oder wässrige Lösungen zum Einsatz. Neben der gebrauchten Waschlösung E2 wird der organische Strom S3 erhalten, der frei von organischem Extraktionsmittel und weitestgehend wassergesättigt ist. Zur Entfernung des homogen gelösten Wassers (als separate Phase vorliegendes Wasser kann einfach, beispielsweise über einen Abscheider, abgetrennt werden) wird der Strom S3 in eine Kolonne K4 (Stufe d) geführt. Die am Kopf der Kolonne K4 anfallenden Brüden werden im Kondensator W8 kondensiert. Dabei werden zwei Phasen erhalten, eine wässrige und eine organische Phase. Die organische Phase wird in die Kolonne K4 als Rücklauf zurückgeführt, die wässrige Phase D4 ausgeschleust. Am Sumpf der Kolonne wird der weitgehend wasserfreie Sumpfstrom S4 erhalten, der in der Oligomerisierung des erfindungsgemäßen Verfahrens (Oligomerisierung eines Teils der enthaltenen Butene) weiter umgesetzt wird.

Die Beheizung der Kolonne K4 erfolgt über den Sumpfverdampfer W7. Als Heizmedium kann beispielsweise Dampf, Kondensat oder Warmwasser eingesetzt werden. Die Beheizung kann auch durch Wärmeintegration mit dem Extraktionsmittelkreislauf erfolgen. Vorzugsweise wird hierfür der Ausgangsstrom des W3 aus **Fig. 1** genutzt, der nicht direkt zum W1, sondern erst über den Wärmetauscher W7 geführt wird (in **Fig. 2** nicht dargestellt).

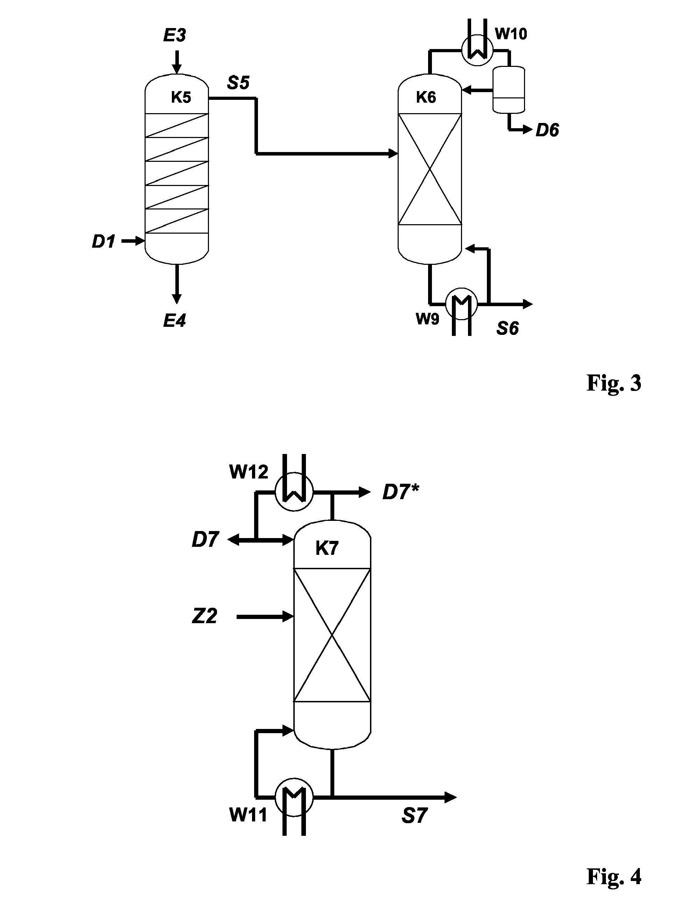

Das Destillat D1 aus der Extraktionskolonne K1 kann auf dieselbe Art aufgereinigt werden wie das Destillat D2 aus Kolonne K2. Eine solche Aufarbeitung ist in **Fig. 3** wiedergegeben. In einer Extraktionskolonne K5 wird das Destillat D1 bzw. der unpolare Teil des Destillats D1 mit einer Waschlösung E3 von organischen Resten des Extraktionsmittels befreit (Stufe e)). Als Waschlösungen kommen Wasser oder wässrige Lösungen zum Einsatz. Neben der gebrauchten Waschlösung E4 wird der organische Strom S5 erhalten, der frei von organischem Extraktionsmittel und weitestgehend wassergesättigt ist. Zur Entfernung des homogen gelösten Wassers (als separate Phase vorliegendes Wasser kann einfach, beispielsweise über einen Abscheider, abgetrennt werden) wird der Strom S5 in eine Kolonne K6 geführt (Stufe f)). Die am Kopf der Kolonne K6 anfallenden Brüden werden im Kondensator W10 kondensiert. Dabei werden zwei Phasen erhalten, eine wässrige und eine organische Phase. Die organische Phase wird in die Kolonne K6 als Rücklauf zurückgeführt, die wässrige Phase D6 ausgeschleust. Am Sumpf der Kolonne wird der weitgehend wasserfreie Sumpfstrom S6 erhalten. Sein Wassergehalt liegt bevorzugt unter 50 ppm, besonders bevorzugt unter 5 ppm Wasser.

Die Beheizung der Kolonne K6 erfolgt über den Sumpfverdampfer W9. Als Heizmedium kann beispielsweise Dampf, Kondensat oder Warmwasser eingesetzt werden. Die Beheizung kann auch durch Wärmeintegration mit dem Extraktionsmittelkreislauf erfolgen. Vorzugsweise wird hierfür der Ausgangsstrom des W3 (aus **Fig. 1)** genutzt, der nicht direkt zum W1, sondern erst über den Wärmetauscher W9 geführt wird.

Sind im ursprünglich eingesetzten Kohlenwasserstoffstrom auch C₅-Kohlenwasserstoffe vorhanden und ist es notwendig, den Anteil an C₅-Kohlenwasserstoffen in den C₄-Kohlenwasserstofffen zu begrenzen, so kann dies in einer zusätzlichen destillativen Reinigung erfolgen. Eine derartige Reinigung ist in **Fig. 4** schematisch wiedergegeben. Der Zulauf Z2 zur Kolonne K7 enthält neben den C₄-Kohlenwasserstoffen im geringen Maß, vorzugsweise unter 5 Massen-%, besonders bevorzugt unter 2 Massen-%, C₅-Kohlenwasserstoffe. Als Sumpfprodukt der Kolonne wir ein Strom S7 abgezogen, in dem die C₅-Kohlenwasserstoffe angereichert sind. Am Kopf der Kolonne werden die Brüden D7* erhalten, die ganz oder teilweise im Kondensator W 12 kondensiert werden. Das dabei erhaltene Destillat D7 wird ganz oder teilweise in die Kolonne K7 als Rücklauf zurückgeführt. Der Teil, der nicht als Rücklauf eingesetzt wird, wird entweder unkondensiert (D7*) oder nach Kondensation (D7) in weitere Verfahrensschritte weitergeleitet.

Die Beheizung der Kolonne K7 erfolgt über den Sumpfverdampfer W11. Als Heizmedium kann beispielsweise Dampf, Kondensat, oder Warmwasser eingesetzt werden. Die Beheizung kann auch durch Wärmeintegration mit dem Extraktionsmittelkreislauf erfolgen. Vorzugsweise wird hierfür der Ausgangsstrom des W3 (siehe **Fig. 1**) genutzt, der nicht direkt zum W1, sondern erst über den Wärmetauscher W7 geführt wird.

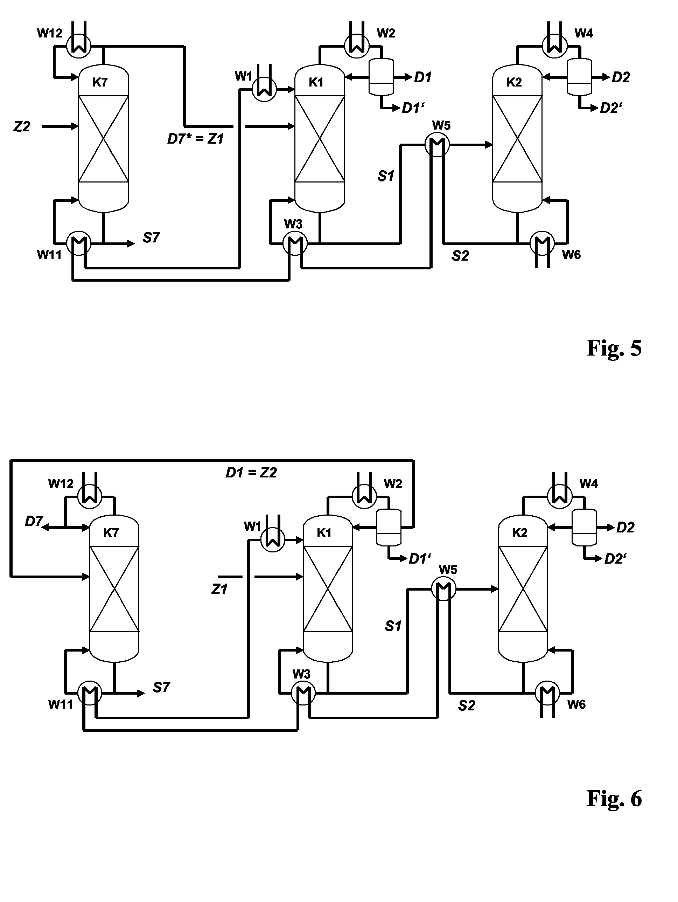

Eine mögliche Verschaltung der Kolonne K7 mit den Kolonnen K1 und K2 ist in **Fig. 5** dargestellt. C₅-Kohlenwasserstoffe werde hierbei vor der Extraktionskolonne K1 abgetrennt. Die im Zulauf Z2 der Kolonne K7 enthaltenen C₅-Kohlenwasserstoffe werden vollständig oder teilweise mit dem Sumpfstrom S7 der Kolonne K7 abgetrennt (Stufe j)). Ein Teil der Brüden der K7 wird im W 12 kondensiert und als Rücklauf in die Kolonne zurückgeführt. Der Rest der Brüden D7* wird direkt der Extraktionskolonne K1 als Zulauf Z1 zugeführt.

Die Beheizung der Kolonne K7 wird durch eine Wärmeintegration mit dem Extraktionsmittelkreislauf realisiert. Der Extraktionsmittelstrom aus W3 wird zur weiteren Ausnutzung der im Extraktionsmittelstrom enthaltenen Wärme über den Wärmetauscher W11 zum W1 geführt. Je nach Anteil an C₅-Kohlenwasserstoffen im Zulauf Z2 und der erforderlichen Abtrenngüte kann es dabei vorkommen, dass die Wärmemenge des Extraktionsmittelstroms hierfür nicht mehr ausreicht. In diesem Fall kann zusätzliche Wärme, beispielsweise über einen zweiten Sumpfverdampfer, in die K7 eingebracht werden. Als Energieträger für den zusätzlichen Wärmetauscher bieten sich Standards wie Dampf, Kondensat oder Warmwasser an.

In **Fig. 6** ist eine alternative Einbindung der C₅-Abtrennung in den Prozess dargestellt. In diesem Fall wird der C₅-haltige Kohlenwasserstoffstrom zuerst in der Extraktionskolonne K1 aufgetrennt. Aus dem Destillat D1 der Kolonne K1, in dem zumindest ein Teil der C₅-Kohlenwasserstoffe enthalten ist, werden dann in der Kolonne K7 die C₅-Kohlenwasserstoffe ganz oder teilweise als Sumpfstrom S7 abgetrennt. Die Wärmeintegration erfolgt analog zu der in **Fig. 5** beschriebenen.

Die beschriebenen Aufreinigung des Stroms D1 und die Abtrennung von C₅-Kohlenwasserstoffen können einzeln oder auch kombiniert zum Einsatz kommen. Ausschlaggebend für den Reinheitsgrad, der erzielt werden soll, ist der weitere Einsatz der Ströme.

Der butenarme Strom D1, der hauptsächlich Butane, Reste an Butenen und gegebenenfalls Reste des Extraktionsmittels enthält, kann für einige Anwendungen direkt eingesetzt werden, beispielsweise als Rohstoff für die Acetylenerzeugung. Durch Hydrierung der noch enthaltenen Olefine zu Alkanen kann n-Butan gewonnen werden, welches in unterschiedlichen Reinheiten beispielsweise als Rohstoff für die Maleinsäureanhydrid-Herstellung oder als Treibgas eingesetzt wird.

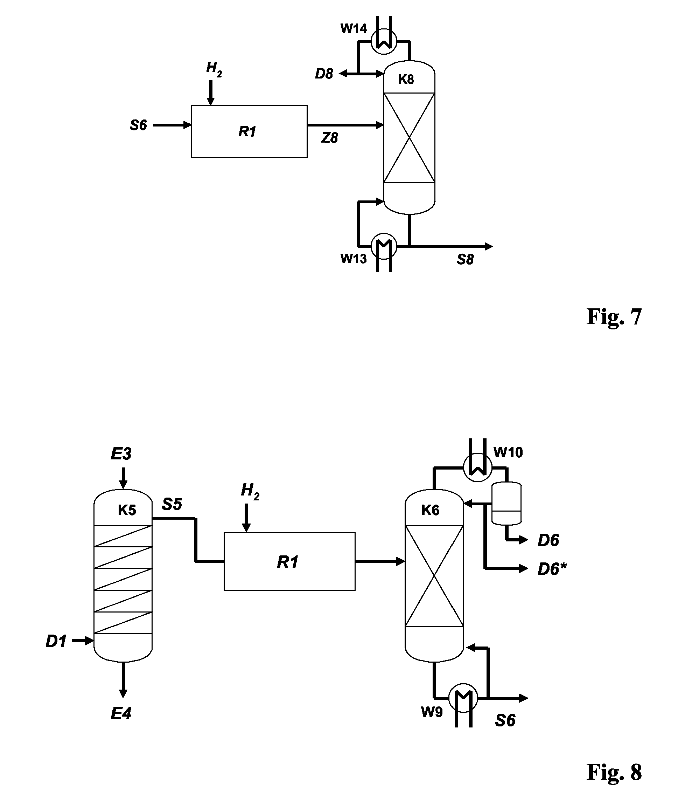

Eine Möglichkeit zur Aufarbeitung des Stroms D1 zu hochreinem n-Butan, das beispielsweise für den Einsatz als Treibgas verwendet wird, ist in **Fig. 7** wiedergegeben. Der über das in Fig. 3 beschriebene Verfahren gereinigte und getrocknete Strom S6 wird einer Hydrierstufe R1 zugeleitet (Stufe i)). Hier werden die restlichen Olefme mit Wasserstoff zu Alkanen umgesetzt. Technische Ausführungen derartiger Hydrierungen sind Stand der Technik. Das aus der Hydrierung erhaltene n-Butan kann zur weiteren Verbesserung der Spezifikation destillativ weiter aufgereinigt werden. Dies kann beispielsweise zur Abtrennung von Anteilen an Isobutan notwendig sein. In **Fig. 7** ist eine derartige Abtrennung durch Kolonne K8 wiedergegeben. Der Austrag der Hydrierung R1 wird als Zulauf Z8 in die Kolonne K8 geleitet. Am Kopf der Kolonne werden Leichtsieder, beispielsweise C₁-C₃ Kohlenwasserstoffe und/oder Isobutan, als Strom D8 im Kondensator W14 auskondensiert und teilweise als Rücklauf in die Kolonne zurückgefahren. Im Sumpf der Kolonne erhält man das aufgereinigte n-Butan als Sumpfstrom S8. Die Beheizung der Kolonne erfolgt über den Sumpfverdampfer W13.

Um aus dem n-Butan noch vorhandene Verunreinigungen im Spurenbereich zu entfernen, kann eine Nachreinigung über Adsorberbetten erfolgen. Diese eignen sich besonders zur Entfernung von geruchsintensivem (Eigengeruch aufweisende) Komponenten, die beispielsweise bei Treibgasen, die in kosmetischen oder pharmazeutischen Produkten eingesetzt werden, störend sind. Ein gebräuchliches Adsorbens ist beispielsweise Aktivkohle.

Eine Alternative zu der in **Fig. 7** aufgezeigten Aufreinigung des n-Butans ist in **Fig. 8** dargestellt. Hier erfolgt die Hydrierung der Restmengen an Olefinen (Stufe i)) nach der Kolonne K5. Da im Zulauf der Hydrierung S5 noch gelöstes Wasser aus der Waschlösung enthalten ist, empfiehlt es sich, die Hydrierung bei Temperaturen zu betreiben, die über der der Extraktion in Kolonne K5 liegen (bevorzugt mindestens 5 °C höher), um das Auftreten von freiem Wasser in der Hydrierung zu vermeiden. Der Austrag der Hydrierung wird dann der Trocknungskolonne K6 zugeleitet. Diese wird wie beschrieben betrieben, mit der Änderung, dass ein Teil der bei der Kondensation im W10 anfallenden organischen Phase D6* nicht in die Kolonne zurückgeführt, sondern ausgeschleust wird.

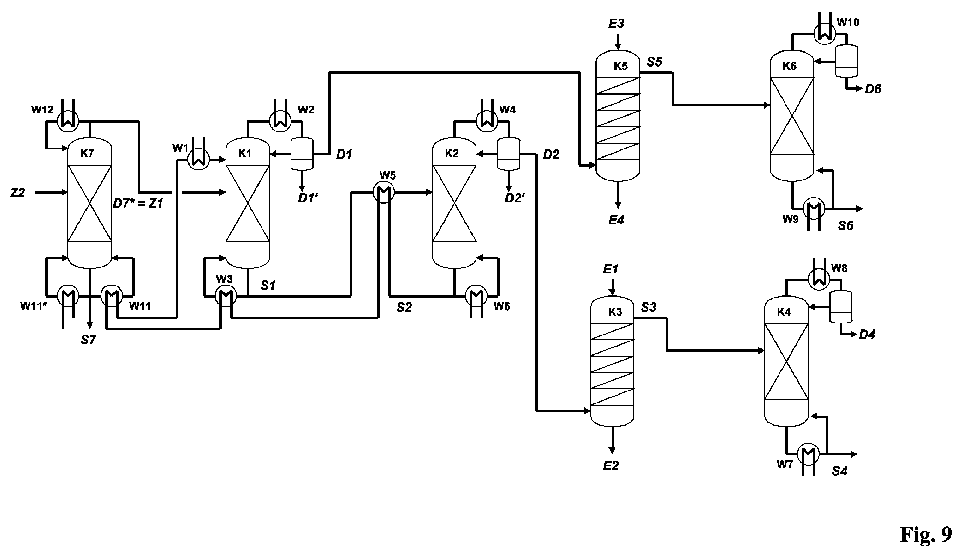

In **Fig. 9** ist eine mögliche Ausführungsform des erfindungsgemäßen Verfahrens dargestellt, die durch Kombination der in den Figuren **Fig. 2, Fig. 3** und **Fig. 5** beschriebenen Anlagen erhalten wird (Stufen a), b), c), d), e), f und j)). Wie in **Fig. 1** sind die Kolonnen K1 und K2 mit Kopfdekantern ausgerüstet. Das kondensierte Kopfprodukt der Kolonnen K1 und K2 wird in den Dekantern in eine polare Phase und eine unpolare Phase getrennt. Die polaren Phasen D1' und D2', die z. B. Reste von Extraktionsmittel aufweisen, können in den Prozess zurückgefahren werden oder einer Aufarbeitung zugeführt werden. Die im Dekanter erhaltenen unpolaren Phasen können teilweise in die jeweilige Kolonne zurückgeführt werden. Die anderen Teile der unpolaren Phasen D1 und D2 werden den Extraktionskolonnen K3 bzw. K5 zugeführt. Zur Aufheizung des Rücklaufs des Sumpfes der Kolonne K7 ist neben dem Wärmetauscher W11, ein Wärmetrauscher W11* vorhanden mit dem zusätzliche Wärme in die K7 eingebracht werden kann, wenn die im Sumpfprodukt S2 der Kolonne K2 vorhandene Wärmeenergie nicht ausreichend ist, um die notwendige Wärmeenergie aufzubringen. Als Energieträger für den zusätzlichen Wärmetauscher können die üblichen Wärmeträger wie z. B. Dampf, Kondensat oder Warmwasser eingesetzt werden.

In **Fig. 10** ist schematisch eine Ausführungsart des erfindungsgemäßen Verfahrens dargestellt, bei der in der Oligomerisierung abgetrennte butenhaltige Ströme in Stufe a) zurückgeführt werden. Der Zulaufstrom Z3, der einen typischen Gehalt von weniger als 50 Massen-% Butenen aufweist, wird hier zusammen mit den butenhaltigen C4-Kohlenwasserstoffen aus der Oligomerisierung als Zulauf Z1 in die Einheit F1 geführt werden. Hier werden die C₄-Kohlenwasserstoffe in einen n-Butan (D1) reichen und einen n-Buten (D2) reichen Strom getrennt (vergleiche Fig. 1). Der Strom D2 wird anschließend in der Einheit F2 (vergleiche Fig. 2) gewaschen und getrocknet. Der hieraus erhaltene Strom von C₄-Kohlenwasserstoffen S4 wird in die Oligomerisierung zurückgeführt. D4 bezeichnet die bei der Trocknung der C₄-Kohlenwasserstoffe erhaltene wässrige Phase. In der Oligomerisierung werden die gebildeten Oligomere von den C₄-Kohlenwasserstoffen abgetrennt, die nicht umgesetzte Butene enthalten.

In **Fig. 11** ist wie in **Fig. 10** eine Ausführungsart des erfindungsgemäßen Verfahrens dargestellt, bei der in der Oligomerisierung abgetrennte butenhaltige Ströme in Stufe a) zurückgeführt werden. Der Unterschied besteht in der Zuführung des Zulaufstroms Z3. Dieser wird zusammen mit S4 in die Oligomerisierung geführt. Der Strom Z3 hat hier einen typischen Gehalt von mehr als 50 Massen-% an Butenen.

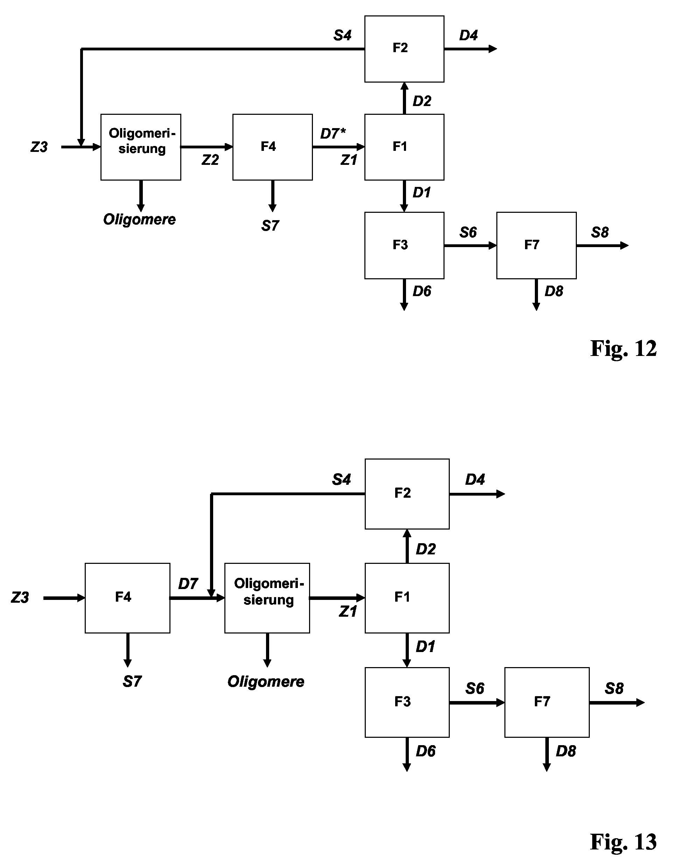

In **Fig. 12** ist schematisch ein Anlagenverbund wiedergegeben, in dem das erfindungsgemäße Verfahren mit einer Einheit zur Herstellung von Oligomeren und von hochreinem n-Butan kombiniert wurde. Um C₅-Kohlenwasserstoffe abzutrennen wurde im Vergleich zu **Fig. 10** zwischen die Oligomerisierung und die Stufe F1 eine Stufe F4 geschaltet (vergleiche **Fig. 5),** in der C₅-Kohlenwasserstoffe abgetrennt werden. Der aus F1 erhaltene Buten-arme Strom D1 wird in der Einheit F3 aufgereinigt (vergleiche **Fig. 3)** und der gereinigte und getrocknete Strom S6 der Einheit F7 (vergleiche **Fig. 7**) zur Hydrierung und Destillation zugeleitet. Aus F7 wird der n-Butan-Strom S8 erhalten, neben den abgetrennten Leichtsiedern D8.

Insbesondere für die optionale Abtrennung der C₅-Kohlenwasserstoffe und der Leichtsieder sind diverse andere Verschaltungen möglich. Für die Wahl der technisch günstigsten Konstellation ist dabei die Zusammensetzung der eingesetzten C₄-Kohlenwasserstoffe ein wesentlicher Faktor. Dabei können auch Spurenkomponenten entscheidend sein. Sind im eingesetzten externen Kohlenwasserstoffstrom Z3 (**Fig. 11)** beispielsweise hochsiedende Verunreinigungen enthalten, die als Moderatoren für die Oligomerisierung wirken, ist es gegebenenfalls sinnvoll, diese zusammen mit C₅-Kohlenwasserstoffen in einer C₅-Abtrennung vor der Oligomerisierung zu entfernen. Die Rückführung des Buten-reichen Stroms S4 erfolgt dann bevorzugt hinter die C₅-Abtrennung. Eine solche Verschaltung ist in **Fig. 13** wiedergegeben.

Das bei der technischen Ausführung von Destillations- und Extraktionskolonnen Einrichtungen für Abgasströme, Pumpen, Ventile etc. vorgesehen werden, gehört zum technischen Grundwissen und wurde daher bei der Beschreibung nicht explizit erwähnt.

**Tabelle 1: Beschreibung der in den Figuren Fig. 1 bis 9 verwendeten Bezeichnungen**

| Bezeichnung | **Beschreibung** |
|---|---|
| (H2) | (Wasserstoff) |
| B1 | Vorrats und Ausgleichsbehälter Extraktionsmittel |
| D1 | Destillat K1 (Buten-armer Strom) |
| D1' | Destillat K1 (wässrige Phase) |
| D2 | Destillat K2 (C4, Buten reich) |
| D2' | Destillat K2 (wässrige Phase) |
| D4 | Destillat K4 (wässrige Phase am Kopf der Trocknungskolonne K4) |
| D4* | Destillat K4 (organische Phase am Kopf der Trocknungskolonne K4) |
| D6 | Destillat K6 (wässrige Phase am Kopf der Trocknungskolonne K6) |
| D6* | Destillat K6 (organische Phase am Kopf der Trocknungskolonne K6) |
| D7 | Destillat der K7 (kondensiert (C₅-abgereichert)) |
| D7* | Brüdenstrom der K7 (C₅-abgereichert) |
| D8 | Destillat der K8 (Leichtsieder) |
| E1 | Waschlösung, frisch |
| E2 | Waschlösung, gebraucht |
| E3 | Waschlösung, frisch |
| E4 | Waschlösung, gebraucht |
| F1 | Einheit in der die Stufen a) und b) ausgeführt werden |
| F2 | Einheit in der die Stufen c) und d) ausgeführt werden |
| F3 | Einheit in der die Stufen e) und f) ausgeführt werden |
| F4 | Einheit in der die Stufe j) durchgeführt wird |
| F7 | Einheit in der die Stufe g) durchgeführt wird |
| K1 | Kolonne 1 (Extraktionskolonne) |
| K2 | Kolonne 2 (Ausgaserkolonne) |
| K3 | Kolonne 3 (Entfernung von Extraktionsmittelresten) |
| K4 | Kolonne 4 (Trocknung des Buten-reichen Stroms) |
| K5 | Kolonne 5 (Trocknung des Buten-armen Stroms) |
| K5 | Kolonne 5 (Entfernung von Extraktionsmittelresten) |
| K7 | Kolonne 7 (Abtrennung C₅-Kohlenwasserstoffe) |
| K8 | Kolonne 8 (Abtrennung von Leichtsiedern) |
| P | Zuführung des Extraktionsmittels zur Stufe a) |
| P1 | Zuführung Extraktionsmittel (Ersatz oder aus Pufferbehälter) |
| P2 | Ausschleusung Extraktionsmittel (zur Aufarbeitung oder zum Pufferbehälter) |
| R1 | Reaktor 1; Hydrierung der Olefine zu Parafmen |
| S1 | Sumpfstrom der K1 (Extraktionsmittel und C₄, Buten-reich) |
| S2 | Sumpfstrom der K2 (Extraktionsmittel) |
| S3 | Ablauf der K3 (Extraktionsmittel frei, wasserhaltig) |
| S4 | Sumpfstrom der K4 (getrockneter, Buten-reicher Strom) |
| S5 | Ablauf der K5 (Extraktionsmittel frei, wasserhaltig) |
| S6 | Sumpfstrom der K6 (getrockneter, Buten-armer Strom) |
| S7 | Sumpfstrom der K7 (C₅-reicher Strom) |
| S8 | Sumpfstrom der K8 (n-Butan, abgereicherter Leichtsiederanteil) |
| W1 | Zulaufkühler der K1 |
| W10 | Kondensator der K6 |
| W11 | Sumpflieizer der K7 |
| W11* | Zusätzl. Sumpfheizer der K7 |
| W12 | Kondensator der K7 |
| W13 | Sumpfheizer der K8 |
| W14 | Kondensator der K8 |
| W2 | Kondensator der K1 |
| W3 | Sumpfheizer der K1 |
| W4 | Kondensator der K2 |
| W5 | Vorwärmer Zulauf zur K2 |
| W6 | Sumpfheizer der K2 |
| W7 | Sumpfheizer der K4 |
| W8 | Kondensator der K4 |
| W9 | Sumpfheizer der K6 |
| Z1 | Kohlenwasserstoffstrom (2) |
| Z2 | Externer Rohstoffstrom, C₄-Kohlenwasserstoffe mit C₅-Anteil |
| Z3 | Externer Kohlenwasserstoffstrom |
| Z8 | Zulauf zur K8 |

In den nachfolgenden Beispielen wird das erfindungsgemäße Verfahren beispielhaft beschrieben, ohne dass die Erfindung auf diese Ausführungsformen beschränkt sein soll.

### Beispiele 1-3

Die Beispiele 1 bis 3 veranschaulichen den Vorteil, der durch die Rückführung des getrockneten, ungesättigte C₄-Kohlenwasserstoffe enthaltenen Stroms in eine Oligomerisierung erzielt wird.

Der schematische Aufbau der Anlage erfolgte entsprechend Fig. 11. Der Zulaufstrom Z1 zur Einheit F1, in der die C₄-Kohlenwasserstoffe in einen n-Butan (D1) reichen und einen n-Buten reichen (D2) Strom getrennt werden, wird erhalten bei der Abtrennung nicht umgesetzter C₄-Kohlenwasserstoffe aus dem Produkt einer Oligomerisierung. Der in F1 erhaltene butenreiche Strom (D2) wird anschließend in der Einheit F2 gewaschen und getrocknet. Der hieraus erhaltene Strom von C₄-Kohlenwasserstoffen S4 wird zusammen mit dem Zulaufstrom Z3 in die Oligomerisierung geführt.

Die Oligomerisierung erfolgt nach bekanntem Stand der Technik an einem heterogenen Nickel-Katalysator (hergestellt analog US 5,169,824, Beispiel 1) in Rohrbündelreaktoren bei einer Reaktionstemperatur von 80 °C in der flüssigen Phase. Die Abtrennung der nicht umgesetzten C₄-Kohlenwasserstoffe erfolgt destillativ.

Die Einheit F1 umfasst die Stufen a) und b) des erfindungsgemäßen Verfahrens. Die extraktive Destillation in Stufe a) erfolgt in einer Kolonne mit 26 theoretischen Böden bei 0,5 MPa. Als Extraktionsmittel wird eine NMP/Wasser-Mischung mit 9 Massen-% Wasser eingesetzt. Die Zulaufmenge an Extraktionsmittel beträgt 200 t/h. Die Sumpffraktion der Stufe a) wird einer zweiten Kolonne, Stufe b), zugeleitet, die 12 theoretische Böden aufweist. Der Zulauf erfolgt in der Mitte der Kolonne auf Boden 6. Der Druck der Ausgasungskolonne liegt bei 0,5 MPa.

Die Beispiele wurden mit der Simulations-Software AspenPlus Version 12.1 der Firma AspenTech berechnet. Die Ergebnisse dieser Berechnungen werden in der Tabelle 2 wiedergegeben.

**Tabelle 2: Auflistung der Ergebnisse der Beispiele 1 bis 3**

| **Beispiel** | | | **1** | **2** | **3** |
|---|---|---|---|---|---|
| **Z3** | | [t/h] | 20 | 20 | 16,7 |
| | 1-Buten | [%] | 24,2% | 24,2% | 24,2% |
| | c-2-Buten | [%] | 18,4% | 18,4% | 18,4% |
| | t-2-Buten | [%] | 38,4% | 38,4% | 38,4% |
| | n-Butan | [%] | 19,0% | 19,0% | 19,0% |
| | | | | | |
| **S4** | | [t/h] | | 5,4 | 3,3 |
| | Butene | [%] | | 85,0% | 85,0% |
| | n-Butan | [%] | | 15,0% | 15,0% |
| | | | | | |
| **Z3+S4** | | [t/h] | 20,0 | 25,4 | 20,0 |
| | | | | | |
| **Z1** | | [t/h] | 7,0 | 11,0 | 7,1 |
| | Butene | [%] | 46,0% | 58,2% | 46,0% |
| | n-Butan | [%] | 54,0% | 41,8% | 54,0% |
| | | | | | |
| **Oligomere** | | [t/h] | 13,0 | 14,4 | 12,9 |
| **D1** | | [t/h] | 7,0 | 5,6 | 3,8 |
| | | | | | |
| **Umsatz Olig. per Pass** | | [%] | 80,2% | 69,3% | 79,0% |
| **Umsatz Gesamt** | | | 80,2% | 88,9% | 95,4% |
| | | | | | |
| | | | Basis | C2 | B1 |

Das Referenzbeispiel 1 zeigt Umsatz und Ausbeute an Oligomeren ohne Nutzung der Einheit F1. Dabei werden 13 t/h des Zielproduktes Oligomere erhalten, die im Zustrom Z3 enthaltenen Butene werden zu 80,2 % umgesetzt. Bei Betrieb entsprechend dem erfindungsgemäßen Verfahren in Beispiel 2 werden aus der gleichen Zulaufmenge Z3 14,4 t/h Oligomere gewonnen, also 1,4 t/h mehr. Alternativ wird die Menge an Zulauf Z3 reduziert, so dass durch Einsatz des erfindungsgemäßen Verfahrens bei deutlich kleinerem Rohstoffeinsatz Z3 durch eine erhebliche Steigerung des Umsatzes (Gesamt) eine nahezu unveränderte Ausbeute an Oligomeren erzielt wird (Beispiel 3)

### Beispiele 4 und 5

Die Beispiele 4 und 5 zeigen den Einfluss der Einheit F2 auf die Aktivität eines heterogenen Nickelkatalysators in der Oligomerisierung auf.

Die Versuche wurden in einer Technikumsanlage durchgeführt. Als Reaktor für die Oligomerisierung wurde ein von Außen temperierbares Rohr mit einem Innendurchmesser von 2 cm und einer Länge von 200 cm genutzt, in das der heterogene Nickelkatalysator eingefüllt wurde. Der Reaktor wurde auf 90 °C temperiert und von oben mit C₄-Kohlenwasserstoffen beschickt. Am Reaktoreingang und Reaktorausgang wurden Proben entnommen, die gaschromatographisch auf ihre Zusammensetzung analysiert wurden. Über die Zusammensetzung der C₄-Kohlenwasserstoffe (n-Butan als interner Standard) wurde der in der Oligomerisierung erreichte Umsatz bestimmt.

Der in den Versuchen eingesetzte Rohstoff stammt aus einer großtechnischen Anlage. In Versuch 4 wurde er nach einer Wasserwäsche und Trocknung in einer Trocknungskolonne praktisch frei von Acetonitril erhalten. In Versuch 5 sind hingegen 2,5 ppm an Acetonitril enthalten.

**Tabelle 3: Auflistung der Ergebnisse der Beispiele 4 und 5**

| **Beispiel** | | | **4** | **5** |
|---|---|---|---|---|
| Zulaufmenge | | [kg/h] | | |
| C₄ Analyse Zulauf | | | | |
| | 1-Buten | [%] | 18,85 | 13,80 |
| | c-Buten | [%] | 16,49 | 20,85 |
| | t-Buten | [%] | 33,63 | 34,20 |
| | n-Butan | [%] | 30,50 | 30,64 |
| | Acetonitril | [ppm] | < N.G. | 2,5 |
| | Rest | [%] | 0,53 | 0,51 |
| | | | | |

| C₄ Ausgangsanalyse | | | | |
|---|---|---|---|---|
| | 1-Buten | [%] | 3,00 | 3,26 |
| | c-Buten | [%] | 15,79 | 19,75 |
| | t-Buten | [%] | 36,02 | 43,00 |
| | n-Butan | [%] | 44,71 | 33,21 |
| | Acetonitril | [ppm] | < N.G. | - |
| | Rest | [%] | 0,48 | 0,78 |
| | | | | |
| Umsatz Butene | | [%] | 45,5 | 11,4 |

| | | | | |
|---|---|---|---|---|
| N.G.: Nachweisgrenze | | | | |

In beiden Versuchen wurde die Reaktion über einen Zeitraum von 72 Stunden analytisch verfolgt. Die in der Tabelle angegebenen Analysen wurden am Ende des Versuchszeitraums erhalten, an dem sich keine wesentlichen Änderungen mehr zeigten. Im Vergleich wird deutlich, dass bei Anwesenheit von Acetonitril der Umsatz deutlich, von 45,5 auf 11,4 %, zurückgeht.

### Beispiele 6 bis 8

An Hand der nachfolgenden Beispiele werden die Vorteile einer Zulaufvorwärmung der Ausgasungskolonne (Stufe b) mit der Wärmeenergie des Sumpfstromes der Stufe b) aufgezeigt. Der Zulaufstrom mit 225 t/h besteht aus einem Gemisch von 89,2 Massen-% NMP, 8,1 Massen-% Wasser und 2,7 Massen-% Butenen. Die Ausgasungskolonne hat 12 theoretische Trennstufen mit Zulaufstufe 6 und wird bei 0,5 MPa betrieben. Die Berechnungen wurden mit der Simulations-Software AspenPlus Version 12.1 der Firma AspenTech berechnet. Das Modell wurde an experimentell ermittelten Phasengleichgewichtsdaten angepasst.

### Beispiel 6 (Vergleichsbeispiel)

In diesem Beispiel wird der Energiebedarf ohne Vorwärmung gezeigt. Der Zulaufstrom der Ausgasungskolonne wird im Siedezustand bei 0,5 MPa in die Kolonne geführt. Die Zulauftemperatur beträgt dabei 125 °C. Das Lösungsmittel wird im Sumpf der Kolonne auf 186,3 °C aufgeheizt. Dazu wird eine Heizdampf-Leistung von 10000 kW benötigt.

### Beispiel 7 (erfindungsgemäß)

Im zweiten Beispiel wird der Zulaufstrom bei 0,5 MPa soweit aufgeheizt, dass sich ein maximaler Wärmeaustausch mit dem Sumpfstrom der Ausgasungskolonne ergibt. Für den Wärmeaustauscher wird eine minimale Temperaturdifferenz von 7 K festgelegt. Hiermit ist eine Vorwärmtemperatur von 168,8 °C erreichbar. Die erforderliche Heizleistung des Verdampfers reduziert sich hierdurch von 10000 kW auf 4152 kW.

### Beispiel 8 (erfindungsgemäß)

Das dritte Beispiel zeigt die Bedingungen, wenn der Zulaufstrom unter Duck vorgewärmt wird, so dass keine Verdampfung im Vorwärmer stattfindet. Hierzu muss der Zulaufstrom auf 1 MPa gebracht werden. Die erforderliche Heizleistung im Verdampfer verändert sich gegenüber Beispiel 2 nicht. Der wesentliche Unterschied zu Beispiel 2 ist eine erhöhte Vorwärmtemperatur von 177,7 statt 168,8 °C.

**Tabelle 4: Vergleich der Temperaturen und der benötigten Heizleistung in den Beispielen 6 bis 8**

| | **Feedstrom** | | | **Sumpfstrom** | | | **Rück-** | **Heizenergie** | |
|---|---|---|---|---|---|---|---|---|---|
| | **Druck** | **Temperatur** | | **Druck** | **Temperatur** | | **lauf** | **Vor-** | **Ver-** |
| | | **Ein** | **Aus** | | **Ein** | **Aus** | | **wärmer**^{**#**} | **dampfer*** |
| | **MPa** | **°C** | **°C** | **MPa** | **°C** | **°C** | **t/h** | **kW** | **kW** |
| **Beispiel 6** | 0,5 | 125,0 | 125,0 | 0,5 | 186,3 | 186,3 | 5,6 | 0 | 10000 |
| **Beispiel 7** | 0,5 | 125,0 | 168,8 | 0,5 | 186,3 | 132,0 | 29,4 | 8288 | 4152 |
| **Beispiel 8** | 1 | 125,0 | 177,7 | 0,5 | 186,3 | 132,0 | 29,4 | 8288 | 4152 |

| | | | | | | | | | |
|---|---|---|---|---|---|---|---|---|---|
| * von außen in den Prozess eingebrachte Heizenergie ^{#} durch Energierückgewinnung in den Prozess eingebrachte Heizenergie | | | | | | | | | |

Wie der Tabelle 1 entnommen werden kann, ist ohne eine Vorwärmung (Beispiel 1 gemäß dem Stand der Technik) eine deutlich höhere Heizleistung im Sumpfverdampfer notwendig, als mit Vorwärmung (Beispiele 2 und 3). Des weiteren ist zu erkennen, dass durch eine partielle Verdampfung des Zulaufstromes (Beispiel 2) eine deutlich kleinere Endtemperatur der Zulaufvorwärmung erreicht werden kann als in Beispiel 3. Hierdurch kann die Austauschfläche des Zulaufvorwärmers (Feedvorwärmers) reduziert werden, da die mittlere Temperaturdifferenz größer wird.

## Patentansprüche

1. Verfahren zur Oligomerisierung von Butenen in Gegenwart eines Übergangsmetallkatalysators, bei dem als Einsatzstoff ein Kohlenwasserstoffstrom eingesetzt wird, der ganz oder teilweise aus einem Kohlenwasserstoffstrom (1) besteht, der überwiegend Butene enthält und der durch Abtrennung aus einem Strom von Kohlenwasserstoffen (2) mit geringerem Gehalt an Butenen erhalten wurde,
**dadurch gekennzeichnet, dass**
der Kohlenwasserstoffstrom (1) durch
a) extraktive Destillation eines gesättigte und ungesättigte C₄-Kohlenwasserstoffe enthaltenden Stroms (2) mit einem polaren Extraktionsmittel unter Erhalt einer an gesättigten Kohlenwasserstoffen angereicherten Kopffraktion (3) und einer an ungesättigten Kohlenwasserstoffen angereicherten und das polare Extraktionsmittel enthaltenden Sumpffraktion (4),
b) destillative Trennung der Sumpffraktion (4) in eine Kopffraktion (5), die als ungesättigte Kohlenwasserstoffe Butene enthält und eine Sumpffraktion (6), die das polare Extraktionsmittel enthält,
c) Waschen zumindest eines Teils der Kopffraktion (5) mit Wasser oder einer wässrigen Lösung und
d) Trocknung des in Schritt c) behandelten Teils der Kopffraktion (5) unter Erhalt eines weitgehend wasserfreien butenhaltigen Kohlenwasserstoffstroms (1),
erhalten wird.

2. Verfahren nach Anspruch 1,
**dadurch gekennzeichnet,**
**dass** in der Oligomerisierung als Übergangsmetallkatalysator ein heterogener Nickelkatalysator eingesetzt wird.

3. Verfahren nach Anspruch 1 oder 2,
**dadurch gekennzeichnet,**
**dass** die Wäsche gemäß Schritt c) mit Wasser oder einer wässrigen Lösung mehrstufig erfolgt.

4. Verfahren nach zumindest einem der Ansprüche 1 bis 3,
**dadurch gekennzeichnet,**
**dass** die Trocknung des unpolaren Stroms gemäß Schritt d) in einer Destillationskolonne erfolgt, bei der der getrocknete Strom als Sumpfprodukt erhalten wird.

5. Verfahren nach zumindest einem der Ansprüche 1 bis 4,
**dadurch gekennzeichnet,**
**dass** in Stufe a) ein Kohlenwasserstoffstrom (2) eingesetzt wird, der als gesättigte Kohlenwasserstoffe n-Butan und optional Isobutan und als ungesättigte Kohlenwasserstoffe 1-Buten, cis-2-Buten und/oder trans-2-Buten aufweist.

6. Verfahren nach zumindest einem der Ansprüche 1 bis 5,
**dadurch gekennzeichnet,**
**dass** in Stufe a) ein Kohlenwasserstoffstrom (2) eingesetzt wird, der zumindest von 20 bis 75 Massen-% Butene aufweist.

7. Verfahren nach zumindest einem der Ansprüche 1 bis 6,
**dadurch gekennzeichnet,**
**dass** in Stufe a) ein Kohlenwasserstoffstrom (2) eingesetzt wird, der zumindest teilweise bei der Aufarbeitung der C₄-Fraktion eines FC-Crackers erhalten wird und/oder der ganz oder teilweise in einer Oligomerisierung durch Abtrennung vom Produkt der Oligomerisierung als Strom erhalten wird, der in der Oligomerisierung nicht umgesetzte C₄-Kohlenwasserstoffe aufweist.

8. Verfahren nach zumindest einem der Ansprüche 1 bis 7,
**dadurch gekennzeichnet,**
**dass** in Stufe a) ein Kohlenwasserstoffstrom (2) eingesetzt wird, der bis zu 5 Massen-% an C₅-Kohlenwasserstoffen aufweist, und dass aus dem Kohlenwasserstoffstrom vor dem Eintritt in die Stufe a) Kohlenwasserstoffe mit einer Anzahl an Kohlenstoffatomen größer 4 teilweise oder vollständig destillativ abgetrennt werden.

9. Verfahren nach zumindest einem der Ansprüche 1 bis 8,
**dadurch gekennzeichnet,**
**dass** in der Oligomerisierung ein Einsatzkohlenwasserstoffstrom eingesetzt wird, der zusätzlich zum Kohlenwasserstoffstrom (1) einen oder mehrere weitere C₄-Kohlenwasserstoffstöme aufweist.

10. Verfahren nach Anspruch 9,
**dadurch gekennzeichnet,**
**dass** der oder die zusätzlich eingesetzten Kohlenwasserstoffströme zumindest 50 Massen-% Butene aufweisen.

11. Verfahren nach Anspruch 9 oder 10,
**dadurch gekennzeichnet,**
**dass** der oder die zusätzlichen Kohlenwasserstoffströme bei der Aufarbeitung eines C₄-Schnittes eines Steamcrackers oder eine FC-Crackers anfallen.

12. Verfahren nach zumindest einem der Ansprüche 1 bis 11,
**dadurch gekennzeichnet,**
**dass** der butenhaltige Kohlenwasserstoffstrom (1) vor der Verwendung als Einsatzstoff in der Oligomerisierung durch in Kontaktbringen mit einem Adsorber nachgereinigt wird.

13. Verfahren nach zumindest einem der Ansprüche 1 bis 12,
**dadurch gekennzeichnet,**
**dass** die Destillation gemäß Stufe a) oder Stufe b) in einer Apparatur durchgeführt wird, die einen Dekanter aufweist, und in diesem Dekanter die kondensierte Kopffraktion aus der Destillation in einen unpolaren Strom, der Kohlenwasserstoffe aufweist, und einen polaren, wässrigen Strom getrennt wird.

14. Verfahren nach Anspruch 13,
**dadurch gekennzeichnet,**
**dass** sowohl die extraktive Destillation in Stufe a) als auch die destillative Trennung in Stufe b) in einer Apparatur durchgeführt wird, die einen Dekanter aufweist.

15. Verfahren nach Anspruch 13 oder 14,
**dadurch gekennzeichnet,**
**dass** der Dekanter in der jeweiligen Apparatur zur Durchführung der Schritte a) und/oder b) in den Destillatvorlagenbehälter der Kolonne integriert ist.

16. Verfahren nach zumindest einem der Ansprüche 13 bis 15,
**dadurch gekennzeichnet,**
**dass** der aus dem Dekanter oder den Dekantem erhaltene polare, wässrige Strom zumindest teilweise in den Prozess zurückgeführt wird.

17. Verfahren nach Anspruch 16,
**dadurch gekennzeichnet,**
**dass** der aus dem Dekanter oder den Dekantem erhaltene polare, wässrige Strom zumindest teilweise dem polaren Extraktionsmittel der Stufe a) zugeführt wird.

18. Verfahren nach zumindest einem der Ansprüche 1 bis 17,
**dadurch gekennzeichnet,**
**dass** die Sumpffraktion aus Stufe b) zumindest teilweise als Extraktionsmittel in die Stufe a) eingespeist wird und dass die in der Sumpffraktion enthaltene Wärmeenergie in einem Wärmetauscher genutzt wird, um den Zulauf zur Destillationskolonne der Stufe b) zu erwärmen.

19. Verfahren nach zumindest einem der Ansprüche 1 bis 18,
**dadurch gekennzeichnet,**
**dass** ein Teil der unkondensierten Kopffraktion der Stufe b) in die Extraktionsstufe a) zurückgeführt wird.

20. Verfahren nach Anspruch 19,
**dadurch gekennzeichnet,**
**dass** zwischen 0 und 51 Massen-% der unkondensierten Kopffraktion der Stufe b) in die Stufe a) zurückgeführt wird.

21. Verfahren nach Anspruch 19 oder 20,
**dadurch gekennzeichnet,**
**dass** der in die Stufe a) zurückgeführte Teil der unkondensierten Kopffraktion b) auf den Betriebsdruck der Stufe a) verdichtet wird.

22. Verfahren nach zumindest einem der Ansprüche 1 bis 21,
**dadurch gekennzeichnet,**
**dass** als polares Extraktionsmittel ein Gemisch von zumindest einem organischen polaren Extraktionsmittel mit 1 bis 20 Massen-% Wasser eingesetzt wird.

23. Verfahren nach zumindest einem der Ansprüche 1 bis 22,
**dadurch gekennzeichnet,**
**dass** als polares organisches Extraktionsmittel Dimethylformamid, N-Methylpyrrolidon, Acetonitril, Furfural, N-Formylmorpholin oder Dimethylacetamid eingesetzt wird.

24. Verfahren nach zumindest einem der Ansprüche 1 bis 23,
**dadurch gekennzeichnet,**
**dass** der Zulauf zur Destillationskolonne gemäß Stufe b) unter einem Druck erwärmt wird, der höher ist als der Druck in der Destillationskolonne b), und der Zulauf nach dem Erwärmen in die Destillationskolonne b) entspannt wird.

25. Verfahren nach zumindest einem der Ansprüche 1 bis 24,
**dadurch gekennzeichnet,**
**dass** der Zulauf zur Destillationskolonne gemäß Stufe b) unter einem Druck erwärmt wird, der dem Druck in der Destillationskolonne b) entspricht.

26. Verfahren nach Anspruch 25,
**dadurch gekennzeichnet,**
**dass** der Zulauf zur Destillationskolonne gemäß Stufe b) vor Eintritt in die Kolonne in einem Kettle-Verdampfer zumindest teilweise verdampft wird.

27. Verfahren nach Anspruch 25 oder 26,
**dadurch gekennzeichnet,**
**dass** der Zulauf zur Destillationskolonne gemäß Stufe b) vor Eintritt in die Kolonne in eine Dampfphase und eine Flüssigphase aufgetrennt wird und diese Phasen einzeln auf unterschiedlichen Böden der Destillationskolonne der Stufe b) eingespeist werden.

28. Verfahren nach zumindest einem der Ansprüche 1 bis 27,
**dadurch gekennzeichnet,**
**dass** der unpolare Strom, der am Kopf der Kolonne der Stufe a) erhalten wird, mit Wasser oder einer wasserhaltigen Lösung gewaschen wird und/oder dass im unpolaren Strom, der am Kopf der Kolonne der Stufe a) erhalten wird, enthaltene Olefine in einer Hydrierstufe zu Alkanen umgesetzt werden und/oder dass der unpolare Strom, der am Kopf der Kolonne der Stufe a) erhalten wird, zu einem wasserfreien Produkt aufgearbeitet wird und/oder dass der unpolare Strom, der am Kopf der Kolonne der Stufe a) erhalten wird, mit einem Adsorber behandelt wird.
